# EUROPEAN PATENT APPLICATION

(11) **EP 4 144 859 A1**
(43) Date of publication of application: **08.03.2023**
(21) Application number: 20932961.4
(22) Date of filing: 29.09.2020
(51) Int. Cl.: C12Q 1/68, C12N 15/11

(54) **TUMOR DETECTION REAGENT AND KIT**

(30) Priority: 30.04.2020 CN 202010361690
(71) Applicant: Creative Biosciences (Guangzhou) Co., Ltd., Guangzhou, Guangdong 510530 (CN)
(72) Inventor: ZHAO, Rongsong, Guangzhou, Guangdong 510530 (CN); HUANG, Longwu, Guangzhou, Guangdong 510530 (CN); ZOU, Hongzhi, Guangzhou, Guangdong 510530 (CN)
(74) Representative: J A Kemp LLP
(86) International application number: PCT/CN2020/118994
(87) International publication number: WO 2021/218031

(57) **Abstract**

Disclosed in the present application are a tumor detection reagent and a kit, comprising a detection reagent for the methylation of a specific nucleic acid fragment, which is used to detect a modified sequence of the specific nucleic acid fragment. The reagent of the present application has passed through experiments and is confirmed to be able to detect and diagnose tumors.

## Description

### TECHINCAL FIELD

The present disclosure belongs to the field of gene detection, and more specifically, the present disclosure relates to a tumor detection reagent and a kit.

### BACKGROUND ART

With the development of social economy, the number of patients suffering from tumors is increasing, and the incidence is becoming younger. Early, painless, rapid and accurate detection of tumors has become an increasingly urgent need.

The existing conventional tumor diagnosis and follow-up methods mainly include *in vitro* imaging, *in vivo* microscopy, tissue biopsy, and exfoliative cytology or excremental cytology. *In vitro* imaging techniques mainly include computed tomography, nuclear magnetic resonance imaging, transabdominal ultrasound, *etc.,* and these techniques often have a high false positive rate in the early diagnosis of cancer. *In vivo* microscopy, as the golden standard for the diagnosis of some tumors, has been improved and uses soft materials in recent years, but it is still highly invasive and brings great pain to patients, who will suffer from pain, bleeding, and other problems within few days after detection. Furthermore, *in vivo* microscopy has low specificity and sensitivity for the early diagnosis of some tumors. Tissue biopsy is used to detect suspected diseased tissues and mainly used to detect the morphology of tumor cells and biomarkers, with high specificity and sensitivity. However, tissue biopsy is somewhat invasive to patients during sampling, and has defects such as long sample pre-treatment time and complicated steps. Exfoliative cytology or excremental cytology is a non-invasive test, and therefore is widely applied to the diagnosis of several tumors. However, some exfoliative cytology or excremental cytology tests cannot exclude the presence of low-grade tumors, and result in low sensitivity. Detection methods, such as nuclear matrix protein-22, tumor-associated antigens, Immuno Cyt assay, and Uro Vysion assay, are not applied to routine clinical testing due to their low sensitivity and/or specificity.

Epigenetics is a rapidly growing field in cancer biology and holds great potential for clinical and translational medicine research. Studies have found that biochemical pathways critical for tumorigenesis are partially regulated by epigenetic phenomena, such as changes in DNA methylation in tumor cells, abnormal histone modifications, miRNA-mediated silencing of various target genes, and reconstruction of hamartomatous nucleosomes. Aberrant DNA methylation is the most extensively studied epigenetic change associated with all types of human cancer. Hypermethylation silencing transcription factors, such as RUNX3, GATA-4, and GATA-5, cause the inactivation of their downstream targets involved in various cellular processes. RUNX3 is an important member of a family of transcription factors, and studies have revealed that in lung cancer cell lines and primary lung cancer specimens, RUNX3 is inactivated by aberrant DNA hypermethylation. Similarly, the GATA family of transcription factors is associated with the pathogenesis of gastrointestinal diseases, and it is observed that among promoters of colorectal cancer, promoter regions of genes GATA-4 and GATA-5 are frequently methylated. Promoters of some genes in bladder cancer are also highly methylated with the frequency of DNA methylation of 48%-96%, which include genes such as A2BP1, NPTX2, POU4F2, HOXA9, MEIS1, GDF15, TMEFF2, VIM, STK11, MSH6, BRCA1, TBX2, TBX3, GATA2, ZIC4, PAX5A, MGMT, and IGSF4^{[1]}.

In recent years, detection of the methylation of a specific region of a tumor-associated gene in a blood, sputum, saliva, faeces or urine sample has been widely applied to aspects such as the diagnosis and early diagnosis of cancer, the prediction of the progress of cancer, the prediction of the prognosis of cancer, post-treatment monitoring, and the prediction of response to anticancer therapy.

### SUMMARY

In some embodiments, the present disclosure provides use of a nucleotide sequence (hereinafter "*nucleotide sequence*" or *"nucleic acid fragment*") that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in preparation of a tumor detection reagent or a kit.

In some embodiments, the present disclosure provides use of a reagent for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in preparation of a tumor detection reagent or a kit.

In some embodiments, the present disclosure also provides a primer, which includes a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 31, SEQ ID NO: 32, SEQ ID NO: 34, SEQ ID NO: 35, SEQ ID NO: 37, SEQ ID NO: 38, SEQ ID NO: 40, SEQ ID NO: 41, SEQ ID NO: 43, SEQ ID NO: 44, SEQ ID NO: 46, or SEQ ID NO: 47, or complementary sequences thereof.

In some embodiments, the primer includes multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of any primer pair shown as SEQ ID NO: 31 and SEQ ID NO: 32, SEQ ID NO: 34 and SEQ ID NO: 35, SEQ ID NO: 37 and SEQ ID NO: 38, SEQ ID NO: 40 and SEQ ID NO: 41, SEQ ID NO: 43 and SEQ ID NO: 44, or SEQ ID NO: 46 and SEQ ID NO: 47.

In some embodiments, the primer includes multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of any primer pair shown as SEQ ID NO: 34 and SEQ ID NO: 35, SEQ ID NO: 43 and SEQ ID NO: 44, or SEQ ID NO: 46 and SEQ ID NO: 47.

In some embodiments, the primer includes multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of a primer pair shown as SEQ ID NO: 43 and SEQ ID NO: 44.

In some embodiments, the present disclosure also provides a probe, which includes a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 33, SEQ ID NO: 36, SEQ ID NO: 39, SEQ ID NO: 42, SEQ ID NO: 45, or SEQ ID NO: 48, or complementary sequences thereof.

In some embodiments, the probe includes a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 36, SEQ ID NO: 45, or SEQ ID NO: 48, or complementary sequences thereof.

In some embodiments, the probe includes a nucleotide sequence that has at least 85% or at least 90% or at least 91% or at least 92% or at least 93% or at least 94% or at least 95% or at least 96% or at least 97% or at least 98% or at least 99% or 100% identity with a sequence shown as SEQ ID NO: 45.

In some embodiments, the primer or probe is an isolated primer or probe.

In some embodiments, the present disclosure also provides use of the abovementioned primer and/or probe in preparation of a tumor detection reagent or a kit.

In the present disclosure, the term "*detection*", is synonymous with *diagnosis,* includes not only early, but also mid and late diagnosis of tumors, and also includes tumor screening, risk assessment, prognosis, disease identification, diagnosis of disease stages, and selection of therapeutic targets.

In an optional embodiment in a disease stage, diagnosis is available by detecting the degree of methylation of the nucleotide sequence in a sample according to the progression of a tumor in different stages or periods. A specific tumor stage of a sample can be detected by comparing the degree of methylation of the nucleotide sequence isolated from tumor samples in different stages to the degree of methylation of the nucleotide sequence of one or more nucleic acids isolated from a sample without abnormal cell proliferation.

In some embodiments, the present disclosure provides a tumor detection reagent, which includes a reagent for detecting a methylation level of a nucleotide sequence that has at least 85% or at least 90% or at least 91% or at least 92% or at least 93% or at least 94% or at least 95% or at least 96% or at least 97% or at least 98% or at least 98% or at least 99% or 100% identity with a nucleotide sequence shown as SEQ ID NO: 4.

Methylation refers to addition of a methyl group to cytosine. After being treated with a hydrosulfite or a bisulfite or a hydrazine salt, cytosine is transformed into uracil. Because uracil is similar to thymine, it is recognized as thymine during PCR amplification. Thus, in a PCR-amplified sequence, unmethylated cytosine is transformed into thymine (C is transformed into T), and methylated cytosine (C) is not transformed. MSP is the commonly used PCR technique for detecting gene methylation, in which primers are designed for a treated methylated fragment (i.e., untransformed C in the fragment), and then PCR amplification is performed. If the fragment is amplified, it is indicated that the fragment is methylated, and if the fragment is unamplified, it is indicated that the fragment is unmethylated.

In some embodiments, the methylation level detection reagent is used to detect a sequence modified with a hydrosulfite or a bisulfite or a hydrazine salt of the nucleotide sequence.

In some embodiments, a sequence modified with a bisulfite of the nucleotide sequence is detected.

In some embodiments, the methylation level detection reagent includes primers and a probe for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4.

In some embodiments, a forward primer of the primers has any one of the following nucleotide sequences:
I. nucleotide sequences that have at least 85% or at least 90% or at least 91% or at least 92% or at least 93% or at least 94% or at least 95% or at least 96% or at least 97% or at least 98% or at least 99% or 100% identity with nucleotide sequences shown as SEQ ID NO: 31, SEQ ID NO: 34, SEQ ID NO: 37, SEQ ID NO: 40, SEQ ID NO: 43, or SEQ ID NO: 46; or
II. complementary sequences of the sequences shown in I.

In some embodiments, a reverse primer of the primers has any one of the following nucleotide sequences:
III. nucleotide sequences that have at least 85% or at least 90% or at least 91% or at least 92% or at least 93% or at least 94% or at least 95% or at least 96% or at least 97% or at least 98% or at least 99% or 100% identity with nucleotide sequences shown as SEQ ID NO: 32, SEQ ID NO: 35, SEQ ID NO: 38, SEQ ID NO: 41, SEQ ID NO: 44, or SEQ ID NO: 47; or
IV complementary sequences of the sequences shown in III.

The primers are used to amplify the nucleotide sequence. It is well known in the art that the successful design of primers is critical for PCR. Compared with common PCR, the design of primers is more critical in methylation level detection. The reason is that methyl sulfurization induces the transformation of "C" in a DNA strand to "U", resulting in reduction of the GC content and the presence of long contiguous "T" in a sequence after PCR. It is easy to cause DNA strand breakage, and thus it is difficult to select stable primers with appropriate Tm values. On the other hand, in order to distinguish sulfurized DNA from unsulfurized and incompletely treated DNA, primers need to have a sufficient number of "C", which increases the difficulty of selecting stable primers. Therefore, in DNA methylation level detection, selection of a fragment to be amplified with primers, such as the length and position of the fragment to be amplified, selection of primers, *etc.* have an influence on the detection sensitivity and specificity. By experiments, the inventors find that different target fragments to be amplified and primers make a difference in detection results. Many times, some genes or nucleotide sequences have been found to be differentially expressed in tumors and non-tumors, but there is a long way to transform these genes into tumor markers and apply the tumor markers clinically. The main reason is the limitation of detection reagents, which makes it difficult for the detection sensitivity and specificity of these potential tumor markers to meet the requirements of detection, or a detection method is complicated in operation and high in cost, and is difficult to be applied clinically on a large scale.

In some embodiments, the probe has any one of the following nucleotide sequences:
V. nucleotide sequences that have at least 85% or at least 90% or at least 91% or at least 92% or at least 93% or at least 94% or at least 95% or at least 96% or at least 97% or at least 98% or at least 99% or 100% identity with nucleotide sequences shown as SEQ ID NO: 33, SEQ ID NO: 36, SEQ ID NO: 39, SEQ ID NO: 42, SEQ ID NO: 45, or SEQ ID NO: 48; or
VI. complementary sequences of the sequences show in V.

In some embodiments, the reagent includes a reagent for detecting a reference gene.

In some embodiments, the reference gene is β-actin (ACTB).

In some embodiments, the reagent for detecting the reference gene is primers and a probe for the reference gene.

In some embodiments, the reagent also includes at least one of a hydrosulfite, a bisulfite, and a hydrazine salt for modifying the nucleotide sequence, and of course, the reagent may not include the hydrosulfite, the bisulfite or the hydrazine salt.

In some embodiments, the reagent includes one or more of a DNA polymerase, dNTPs, Mg²⁺ ions, and a buffer; and optionally, the reagent includes a PCR reaction system that includes a DNA polymerase, dNTPs, Mg²⁺ ions, and a buffer and is used for amplifying a modified nucleotide sequence.

A sample to be detected with the detection/diagnosis reagent of the present disclosure may be selected from at least one of tissue, body fluid, and excrement.

Optionally, the tissue is bladder tissue.

Optionally, the body fluid is at least one of blood, serum, plasma, extracellular fluid, tissue fluid, lymph fluid, a cerebrospinal fluid, and an aqueous humour.

Optionally, the excrement is selected from at least one of sputum, urine, saliva, and faeces.

Optionally, the excrement is selected from urine.

In some embodiments, the present disclosure also provides a kit, which includes the above tumor detection reagent. In some embodiments, the kit also includes instructions. In some embodiments, the kit also includes a nucleic acid extraction reagent. In some embodiments, the kit also includes a sampling apparatus.

In some embodiments, tissue to be detected with the detection reagent of the present disclosure is selected from tumor tissue and para-carcinoma normal tissue (or benign tumor tissue).

In some embodiments, the present disclosure also provides a method for detecting a methylation level of the nucleotide sequence in a sample, characterized by including the following steps: (1) treating a sample to be detected with a hydrosulfite, a bisulfite, and a hydrazine salt to obtain a modified sample to be detected; (2) detecting a methylation level of the nucleotide sequence, for example, using the above reagent or kit to detect the methylation of the nucleotide sequence in the sample to be detected that is modified at step (1). In an optional embodiment, at step (2), real-time fluorescence quantitative methylation-specific polymerase chain reaction is used for detection.

In some embodiment, the present disclosure also provides a tumor detection method, which includes: detecting a methylation level of a nucleotide sequence in a sample to be detected, optionally, by the above method for detecting a methylation level of a nucleotide sequence; and indicating whether a subject has or is at risk of having a tumor according to the deviation, optionally, a methylation level difference, of the methylation level detected, optionally, by the above method for detecting a methylation level of a nucleotide sequence, from a corresponding methylation level in a normal control sample. The term *"deviation"* in the above steps refers to the deviation of a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4.

In some embodiments, the present disclosure also provides a method for treating a tumor in a subject, which includes: detecting a tumor in a subject, including detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in a sample to be detected from the subject, and treating the tumor in a case that the detection of the tumor in the subject indicates that the subject has or is at risk of having the tumor. Optionally, the methylation level is detected by the above method for detecting a methylation level of a nucleotide sequence. Optionally, the tumor in the subject is detected by the above tumor detection method. Optionally, the treatment is the administration of surgery, chemotherapy, radiotherapy, chemoradiotherapy, immunotherapy, oncolytic virus therapy, any other kind of tumor treatment method used in the art, or a combination of these treatment methods.

In some embodiments, the present disclosure also provides a method for designing primers, which includes steps of designing primers for a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4.

In some embodiments, the present disclosure also provides a system for designing primers, which includes:
1) an input component; 2) a processing component; and 3) an output component. Optionally, the input component is configured to read a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4. Optionally, the processing component is loaded with a program for designing primers based on information read by the input component. Optionally, the output component is configured to output primer sequences designed by the processing component.

In some embodiments, the present disclosure also provides a tumor detection system. The system includes: (1) a component for detecting a methylation level of a nucleotide sequence that at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4, and (2) a result determination system.

In some embodiments, the methylation level detection component includes the above detection reagent or kit.

In some embodiments, the result determination component is configured to output the risk of having a tumor and/or a tumor type according to a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 that is detected by the detection system.

In some embodiments, the risk of having a tumor is determined by comparing a methylation level of a sample to be detected to a methylation level of a normal sample, and it is determined that the sample to be detected has a high risk of having a tumor if there is a significant difference or an extremely significant difference between the methylation level of the sample to be detected and the methylation level of the normal sample.

In some embodiments, if the methylation of the nucleotide sequence is positive, it is indicated that a donor of the sample to be detected is a patient with a tumor or at high risk of having a tumor. In an optional embodiment, the positive refers to that when a detection result of a sample to be detected is compared to a detection result of a normal sample, if there is a significant difference or an extremely significant difference between an amplification result of the sample to be detected and an amplification result of the normal sample, a donor of the sample to be detected is positive.

In some embodiments, determination criteria of the determination system include: whether a specimen is a tumor specimen or a normal specimen is determined according to a cut-off value.

In some embodiments, the tumor is selected from urothelial tumors. Optionally, the tumor is selected from bladder cancer, ureteral cancer, renal pelvis cancer, and urethral cancer. Optionally, the tumor is selected from bladder cancer.

The detection method of this application can be used before and after tumor treatment or used in combination with tumor treatment. After treatment, the detection method is used to, for example, evaluate whether the treatment is successful, monitor the relief, relapse and/or progress (including metastasis) of tumors after treatment.

### BRIEF DESCRIPTION OF THE DRAWINGS

Figs. 1A to 1F respectively show ROC curves of detection of nucleic acid fragments 1 to 6 in 108 clinic tissue specimens (including 66 bladder cancer tissue specimens and 42 bladder cancer para-carcinoma tissue specimen);
Fig. 2 shows ROC curves of detection of the nucleic acid fragment 4 and MEIS1 in 97 urine samples (including 45 bladder cancer samples and 52 control samples);

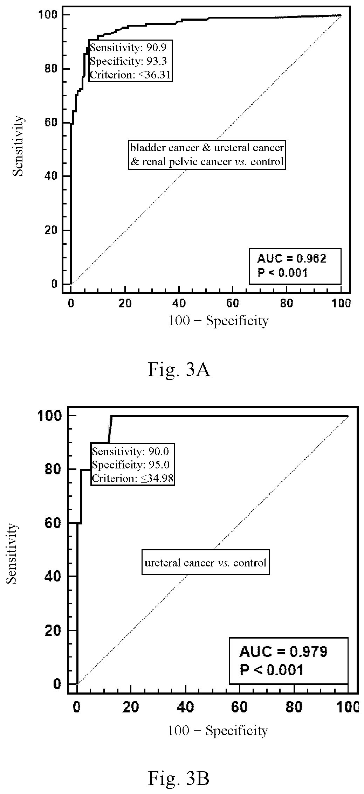

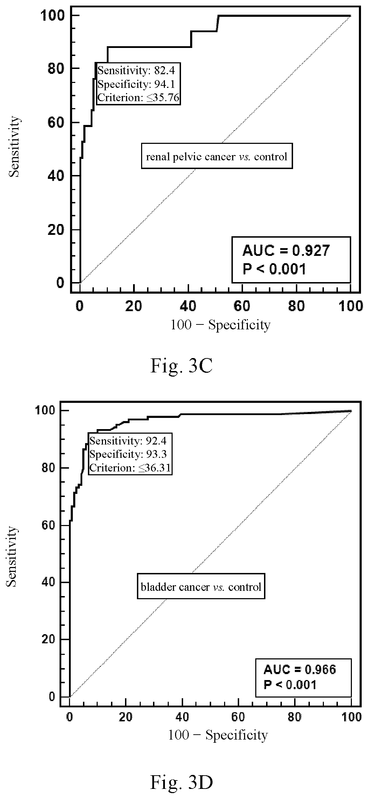

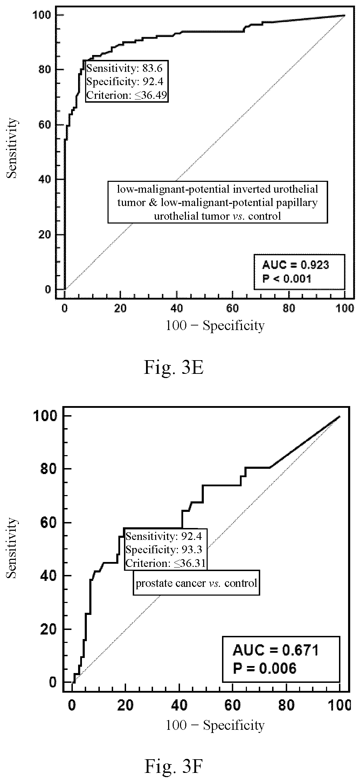

Fig. 3 shows statistical results of detection of the nucleic acid fragment 4 in different types of tumor samples including 299 urine samples (including 1 low-malignant-potential inverted urothelial tumor sample, 16 low-malignant-potential papillary urothelial tumor samples, 105 bladder cancer samples, 31 prostate cancer samples, 17 renal pelvic cancer samples, 10 ureteral cancer samples, and 119 control samples) of different types of tumors with the nucleic acid fragment 4, wherein Fig. 3A shows comparison of ROC curves of a control group and a "bladder cancer & ureteral cancer & renal pelvic cancer" group; Fig. 3B shows comparison of ROC curves of a control group and a ureteral cancer group; Fig. 3C shows comparison of ROC curves of a control group and a renal pelvic cancer group; Fig. 3D shows comparison of ROC curves of a control group and a bladder cancer group; Fig. 3E shows comparison of ROC curves of a control group and a "low-malignant-potential inverted urothelial tumor & low-malignant-potential papillary urothelial tumor" group; and Fig. 3F shows comparison of ROC curves of a control group and a prostate cancer group;
Fig. 4 shows amplification curves and melting curves of different primer and probe sets; and
Fig. 5 shows ROC curves of detection of CG441 in 193 urine samples.

### DETAILED DESCRIPTION

The technical solutions of the present disclosure will be further described below with reference to specific examples, and the specific examples are not intended to limit the scope of protection of the present disclosure. Some nonessential modifications and adjustments made by others on the basis of the idea of the present disclosure shall still fall within the scope of protection of the present disclosure.

In this application, a "*primer*" or a "*probe*" refers to an oligonucleotide, which includes a region complementary to a sequence of at least 6 contiguous nucleotides in a target molecule (e.g., a target nucleic acid fragment). In some embodiments, at least a portion of the primer or probe sequence is not complementary to an amplified sequence. In some embodiments, the primer or probe includes a region complementary to a sequence of at least 9, at least 10, at least 11, at least 12, at least 13, at least 14, at least 15, at least 16, at least 17, at least 18, at least 19 or at least 20 contiguous nucleotides in the target molecule. In a case that the primer or probe includes a region "complementary to at least *x* contiguous nucleotides in the target molecule", the primer or probe is at least 95% complementary to at least *x* contiguous or discontiguous block nucleotides in the target molecule. In some embodiments, the primer or probe is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% complementary to the target molecule.

In this application, a "*normal*" sample refers to the same type of sample isolated from an individual known to be free of the cancer or tumor.

In this application, samples for methylation level detection include, but are not limited to, DNA, RNA, mRNA-containing DNA and RNA samples, and DNA-RNA hybrids. DNA or RNA may be single-stranded or double-stranded.

In this application, a "*subject*" is a mammal, such as a human.

In this application, the term "*methylation level*" is synonymous with "the degree of methylation", and is usually expressed as the percentage of methylated cytosine, which is calculated by the number of methylated cytosine divided by the sum of the number of methylated cytosine and the number of unmethylated cytosine. At present, a methylation level is generally calculated by the number of methylated target genes divided by the number of reference genes, or calculated by other formulas in other prior arts.

In this application, the term "*sample*" is synonymous with *"specimen".*

As used in this application, the term *"and*/*or"* refers to and covers any and all possible combinations of one or more of the associated listed items. When used in a list of two or more items, the term *"and*/*or"* means that any one of the listed items can be used alone or any combination of two or more of the listed items can be used. For example, if a composition, combination, construction, *etc.* is described as including (or containing) components A, B, C and/or D, the composition may include A alone; include B alone; include C alone; include D alone ; include a combination of A and B; include a combination of A and C; include a combination of A and D; include a combination of B and C; include a combination of B and D; include a combination of C and D; include a combination of A, B, and C; include a combination of A, B, and D; include a combination of A, C, and D; include a combination of B, C, and D; or include a combination of A, B, C, and D.

### Example 1

The inventors screened hundreds of gene markers and nucleic acid fragments to study the distribution of methylated sites of genes.

Six nucleic acid fragments screened out by the inventors and screening results are listed herein.

Sequences of nucleic acid fragments 1 to 6 are respectively shown as SEQ ID NO: 1 to SEQ ID NO: 6.

Experimental process:
UM-UC-3, J82, SW780, T24, RT4, 5637, SCaBER, UM-UC-3, and J82 cell lines were obtained from the National Infrastructure of Cell Line Resource, and other cell lines were purchased from ATCC. All the cell lines were resuscitated in recommended media. All the cell lines were negative in mycoplasma detection, and had normal cell morphology. After being expanded, cells were collected, subpackaged at a density of 5×10⁶ cells/tube, and stored in a cryogenic refrigerator at -80°C for DNA extraction.

### 1) DNA extraction

DNA was extracted from 17 bladder cancer cell lines by using DNA extraction kits (QIAGEN DNA Mini Kit, #51306) purchased from QIAGEN.

### 2) DNA modification

The DNA was modified with a sulfite by using a DNA transformation kit (EZ DNA Methylation Kit, D5002) purchased from ZYMO RESEARCH.

The modified sequences are shown in Table 1.

**Table 1**

| Original fragment | After modification |
|---|---|
| Nucleic acid fragment 1 (SEQ ID NO: 1) | SEQ ID NO: 25 |
| Nucleic acid fragment 2 (SEQ ID NO: 2) | SEQ ID NO: 26 |
| Nucleic acid fragment 3 (SEQ ID NO: 3) | SEQ ID NO: 27 |
| Nucleic acid fragment 4 (SEQ ID NO: 4) | SEQ ID NO: 28 |
| Nucleic acid fragment 5 (SEQ ID NO: 5) | SEQ ID NO: 29 |
| Nucleic acid fragment 6 (SEQ ID NO: 6) | SEQ ID NO: 30 |

### 3) Amplification and detection

Primers and probes for determining whether the nucleic acid fragments are methylated are shown in Table 2.

**Table 2 Primer and probe sequences for nucleic acid fragments**

| **Nucleic acid fragment** | **Primer and probe sequences** |
|---|---|
| ACTB (reference gene) | F: TTTTGGATTGTGAATTTGTG (SEQ ID NO: 49) |
| | R: AAACCTACTCCTCCCTTAAA (SEQ ID NO: 50) |
| | P: TTGTGTGTTGGGTGGTGGTT (SEQ ID NO: 51) |
| Nucleic acid fragment 1 | F: CGAGAGGTTATATAAGTTTACG (SEQ ID NO: 7) |
| | R: AATTTCCTAACAAATAATTTCCG (SEQ ID NO: 8) |
| | P: CGCCCGAAAACGTATAAATTACTCC (SEQ ID NO: 9) |
| Nucleic acid fragment 2 | F: TTTAAGGATTAATAATAGAGCG (SEQ ID NO: 10) |
| | R: GAACCGCGTAATTAAAAC (SEQ ID NO: 11) |
| | P: CTAACTCTCGCCGTACCGAATC (SEQ ID NO: 12) |
| Nucleic acid fragment 3 | F: GGACGGAGATATAAGGAT (SEQ ID NO: 13) |
| | R: ATAAACGAACACCAAAATC (SEQ ID NO: 14) |
| | P: CGCAACCCCATAAAACCCAA (SEQ ID NO: 15) |
| Nucleic acid fragment 4 | F: GTAGTCGAGAAGTATTCG (SEQ ID NO: 16) |
| | R: GATAAACGTACACTTAACG (SEQ ID NO: 17) |
| | P: ATCCTTCCAACGACAATAACG (SEQ ID NO: 18) |
| Nucleic acid fragment 5 | F: TTCGTTTATTTATGGATTACG (SEQ ID NO: 19) |
| | R: CGTAAATCGACTCCTTATAC (SEQ ID NO: 20) |
| | P: AACAACGACGACCGAACGAT (SEQ ID NO: 21) |
| Nucleic acid fragment 6 | F: GAAAATAATGTCGAATTTCGT (SEQ ID NO: 22) |
| | R: AAAATCGAAAATCCCACG (SEQ ID NO: 23) |
| | P: CTCGAACAAACGTCTCCTTAAC (SEQ ID NO: 24) |

A reaction system is shown in Table 3.

**Table 3**

| **Reaction component** | **Addition amount (µL)** |
|---|---|
| iTaq Universal SYBR Green supermix (2×) | 10 |
| Primers (F+R, 12.5 µM) | 0.2 |
| Nuclease-free Water | 8.8 |
| Template DNA | 1 |
| Total volume | 20 |

An amplification protocol is shown in Table 4.

**Table 4**

| **Step** | **Temperature and time** | **Number of cycles** |
|---|---|---|
| Pre-denaturation | 95°C for 10 min | 1 |
| Amplification | 95°C for 30 s | 45 |
| | 55°C for 30 s | |
| | 72°C for 30 s, collect fluorescence | |
| Melting curve | 95°C for 5 s | 1 |
| | 65°C for 60 s, continuously collect fluorescence until 97°C | |
| Cooling | 40°C for 3 min | 1 |

After detection was completed, the number of copies of each nucleic acid fragment quantitative system in each cell line was quantitatively calculated by using the standard curve, and the degree of methylation of each nucleic acid fragment in 17 bladder cancer cell lines was calculated by a formula of "the number of copies of a nucleic acid fragment ÷ the number of copies of reference gene ACTB × 100".

Methylation levels of the 6 nucleic acid fragments in the 17 bladder cancer cell lines were detected. Detection results are shown in Table 5.

**Table 5 Detection results of methylation levels of nucleic acid fragments in 17 bladder cancer cell lines**

| Cell line | Nucleic acid fragment 1 | Nucleic acid fragment 2 | Nucleic acid fragment 3 | Nucleic acid fragment 4 | Nucleic acid fragment 5 | Nucleic acid fragment 6 |
|---|---|---|---|---|---|---|
| BFTC-905 | 14% | 34% | 56% | 58% | ***73%*** | 55% |
| CAL-29 | 57% | 59% | ***81%*** | 51% | 39% | 50% |
| SCABER | 10% | 8% | 3% | ***90%*** | ***83%*** | ***95%*** |
| VM-CUB-1 | ***88%*** | ***87%*** | ***87%*** | ***87%*** | 56% | ***97%*** |
| RT-112 | ***78%*** | ***71%*** | ***83%*** | ***76%*** | ***66%*** | ***84%*** |
| SW780 | 44% | 51% | ***62%*** | ***72%*** | ***75%*** | ***80%*** |
| DSH1 | ***89%*** | ***85%*** | ***78%*** | ***92%*** | 54% | ***95%*** |
| RT4 | ***90%*** | ***83%*** | ***86%*** | ***74%*** | ***80%*** | ***92%*** |
| SW1710 | ***86%*** | ***67%*** | ***81%*** | ***70%*** | 58% | **77%** |
| 639-V | 35% | ***62%*** | ***91%*** | ***85%*** | ***76%*** | ***95%*** |
| UM-UC-3 | ***92%*** | ***83%*** | ***85%*** | ***89%*** | ***88%*** | ***84%*** |
| LB831-BLC | ***78%*** | ***81%*** | ***89%*** | ***82%*** | ***77%*** | ***72%*** |
| KU-19-19 | 56% | ***68%*** | ***84%*** | ***60%*** | ***63%*** | 34% |
| 647-V | 13% | 50% | ***83%*** | ***83%*** | ***60%*** | ***85%*** |
| 5637 | 1% | 5% | 36% | ***84%*** | ***86%*** | ***98%*** |
| **J**82 | ***79%*** | ***80%*** | ***76%*** | ***74%*** | 35% | ***80%*** |
| T-24 | ***62%*** | ***83%*** | ***87%*** | ***76%*** | 55% | ***83%*** |

It can be seen from Table 5 that in the 17 bladder cancer cell lines, the 6 nucleic acid fragments are methylated to different extents, among which, the nucleic acid fragments 3, 4, and 6 are methylated in more cell lines compared with other fragments.

### Example 2

The sensitivity and the specificity of each nucleic acid fragment of Example 1, to 108 clinic tissue specimens (including 66 bladder cancer tissue specimens and 42 bladder cancer para-carcinoma tissue specimens), were detected.

### Experimental process:

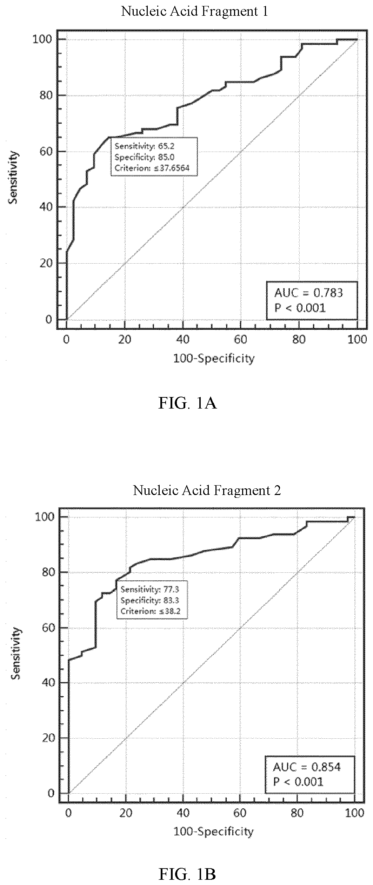

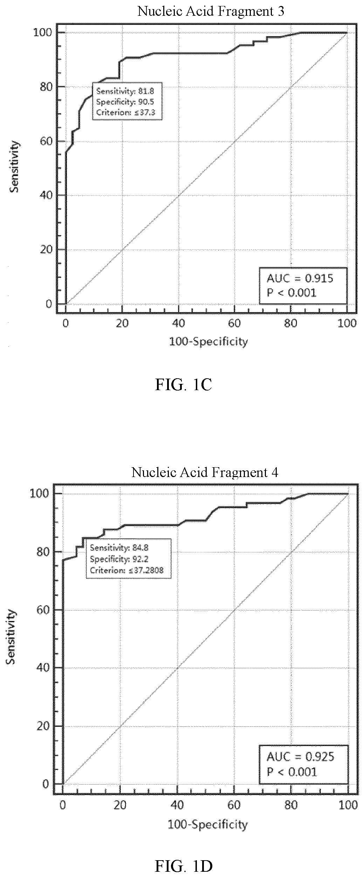

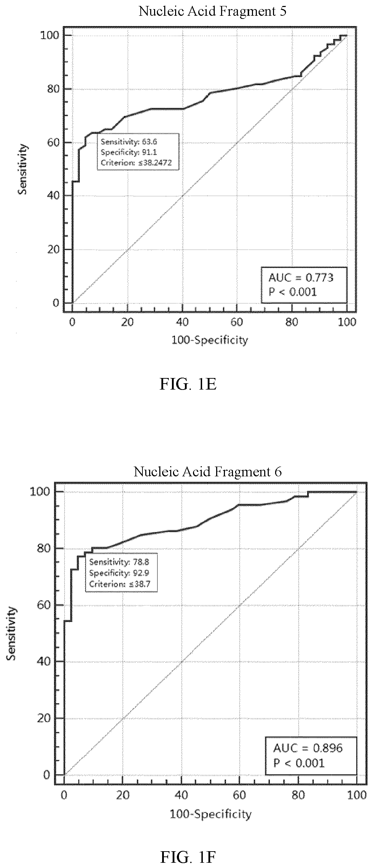

1) DNA extraction
DNA was extracted from each tissue slice specimen by using a DNA extraction kit (HiPure FFPE DNA Kit, D3126-03) purchased from Magen.
2) DNA modification, amplification, and detection, and primers and probes for the fragments were the same as those in Example 1.
3) Calculation method
After detection was completed, the number of copies of each nucleic acid fragment quantitative system in each cell line was calculated by using the standard curve, and the degree of methylation of each nucleic acid fragment in each tissue specimen was calculated by a formula of *"the number of copies of a nucleic acid fragment* ÷ *the number of copies of reference gene ACTB* × *100".* Finally, a threshold was selected as a criterion for distinguishing a cancer group from a control group. If a converted ratio is greater than the set threshold, the methylation is determined as positive, and if the converted ratio is equal to or less than the set threshold, the methylation is determined as negative. According to the criterion, statistical results of detection of the 6 nucleic acid fragments in the 108 clinic tissue samples are shown in Table 6, and ROC curves are shown in Fig. 1A to Fig. 1F.

**Table 6 Detection results of 6 nucleic acid fragments in clinic tissue samples**

| **Fragment** | **Sensitivity** | **Specificity** | **AUC (P<0.001)** |
|---|---|---|---|
| Nucleic acid fragment 1 | 65.2 | 80.5 | 0.783 |
| Nucleic acid fragment 2 | 77.3 | 83.3 | 0.854 |
| Nucleic acid fragment 3 | ***81.8*** | ***90.5*** | ***0.915*** |
| Nucleic acid fragment 4 | ***84.8*** | ***92.2*** | ***0.925*** |
| Nucleic acid fragment 5 | 63.6 | 91.1 | 0.773 |
| Nucleic acid fragment 6 | 78.8 | 92.9 | 0.896 |

The results show that different fragments have differences in the sensitivity and the specificity to the tissue samples. Among them, the sensitivity and the specificity of the nucleic acid fragments 3 and 4 are higher than those of other fragments.

### Example 3

The nucleic acid fragment 4 of the present disclosure was compared to bladder cancer gene methylation markers MEIS1, NKX6-2, OTX1, SIM2, SOX1, BARHL2, ZNF154, and RUNX3 that were often reported in documents.

Twenty paraffin-embedded bladder tissue samples including 10 bladder cancer samples and 10 cystitis glandularis control samples were taken.

### Experimental process:

### 1) DNA extraction

DNA was extracted from each tissue slice by using a DNA extraction kit (HiPure FFPE DNA Kit, D3126-03) purchased from Magen.

### 2) DNA modification

The DNA was modified with a sulfite by using a DNA transformation kit (EZ DNA Methylation Kit, D5002) purchased from ZYMO RESEARCH.

### 3) Amplification and detection

A reaction system and an amplification protocol were the same as those in Example 1.

Primer sequences are shown in Table 7.

**Table 7**

| **Name** | **Sequence** |
|---|---|
| ACTB (reference gene) | Forward primer: TGGTGATGGAGGAGGTTTAGTAAGT (SEQ ID NO: 52) |
| | Reverse primer: AACCAATAAAACCTACTCCTCCCTTAA (SEQ ID NO: 53) |
| Nucleic acid fragment 4 | Forward primer: GCGTTCGGAGTTGTTTAGC (SEQ ID NO: 54) |
| | Reverse primer: CACCGACGCCACAAACG (SEQ ID NO: 55) |
| MEIS 1 | Forward primer: GTTCGGGATAAGATTTCGGGG (SEQ ID NO: 56) |
| | Reverse primer: TAATTAAAACTACGCAACCCGACT (SEQ ID NO: 57) |
| NKX6-2 | Forward primer: AGAAGAAGTATTCGCGTTCGAT (SEQ ID NO: 58) |
| | Reverse primer: GATCATACCCAACGAATAAACG (SEQ ID NO: 59) |
| OTX1 | Forward primer: GTTAGTAGTAGTAGAGCGGGAGC (SEQ ID NO: 60) |
| | Reverse primer: GACGTAAATTAACCACTACTTTCG (SEQ ID NO: 61) |
| SIM2 | Forward primer: GTAGGCGTAGAGGGGATAATTCG (SEQ ID NO: 62) |
| | Reverse primer: ACCCCGCGCTAAATCTACAAC (SEQ ID NO: 63) |
| SOX1 | Forward primer: TAATTAGGATCGGGTTAAACGGT (SEQ ID NO: 64) |
| | Reverse primer: AAACGCTTACTAATCTCCGAAT (SEQ ID NO: 65) |
| BARHL2 | Forward primer: CGTTAGTAGTCGGATTATAAGCGAAC (SEQ ID NO: 66) |
| | Reverse primer: AAAAATTACGAAACAAACACGACCG (SEQ ID NO: 67) |
| ZNF154 | Forward primer: GTTAGGTTTGGGATAGGGATCG (SEQ ID NO: 68) |
| | Reverse primer: CGCTACCATCAAACTCTACG (SEQ ID NO: 69) |
| RUNX3 | Forward primer: GAGGTTTAGTACGCGTTCG (SEQ ID NO: 70) |
| | Reverse primer: CCCGCCTCCTAAATCTATCG (SEQ ID NO: 71) |

### 4) A calculation method was the same as that in Example 2.

Detection results of the nucleic acid fragment 4 and the markers in the 20 tissue samples are shown in Table 8, and statistical analysis results are shown in Table 9.

**Table 8 Detection results of methylation levels of 9 genes in bladder tissue specimens**

| Sample No. | Sample type | MEIS1 | NKX6-2 | OTX1 | SIM2 | SOX1 | BARHL2 | Nucleic acid fragment 4 | ZNF154 | RUNX3 |
|---|---|---|---|---|---|---|---|---|---|---|
| BA01 | bladder cancer | 54.1% | 0.0% | 3.7% | 39.8% | 0.0% | 0.0% | 21.6% | 0.0% | 0.0% |
| BA02 | bladder cancer | **133.0%** | 0.0% | 0.9% | 68.5% | 9.7% | 1.3% | 1.7% | 0.0% | 11.0% |
| BA03 | bladder cancer | 87.5% | 0.0% | 50.6% | 2.6% | 19.4% | 34.8% | 46.8% | 75.8% | 23.0% |
| BA04 | bladder cancer | 8.6% | 36.4% | 46.5% | 48.6% | 66.7% | 36.7% | 46.1% | 79.9% | 37.8% |
| BA05 | bladder cancer | **98.6%** | 0.0% | 0.6% | 28.8% | 6.0% | 0.0% | **0.0%** | 7.7% | 8.1% |
| BA06 | bladder cancer | 52.5% | 21.5% | 53.2% | 60.4% | 8.1% | 20.3% | 45.7% | 87.9% | 3.2% |
| BA07 | bladder cancer | 0.0% | 17.7% | 119.5% | 57.5% | 25.8% | 70.8% | 12.7% | 161.5% | 23.4% |
| BA08 | bladder cancer | 53.8% | 57.7% | 48.5% | 64.7% | 14.1% | 54.8% | 29.0% | 94.0% | 43.4% |
| BA09 | bladder cancer | 54.1% | 21.1% | 47.5% | 28.4% | 8.9% | 23.8% | 43.6% | 55.9% | 55.9% |
| BA10 | bladder cancer | 74.1% | 2.3% | 63.9% | 59.0% | 52.8% | 67.1% | 42.8% | 76.8% | 85.8% |
| BB01 | control | 3.4% | 0.0% | 1.2% | 26.4% | 11.4% | 0.0% | 1.4% | 11.5% | 42.3% |
| BB02 | control | 5.3% | 2.4% | 1.1% | 11.1% | 6.7% | 8.9% | 0.7% | 7.0% | 54.3% |
| BB03 | control | 0.4% | 0.0% | 2.2% | 7.3% | 3.7% | 0.6% | 0.0% | 3.9% | 37.7% |
| BB04 | control | 0.9% | 0.0% | 7.1% | 2.1% | 12.8% | 0.0% | 0.0% | 23.3% | 19.6% |
| BB05 | control | 0.7% | 0.0% | 0.6% | 4.7% | 10.0% | 0.9% | 1.2% | 18.0% | 14.2% |
| BB06 | control | 3.2% | 1.4% | 2.0% | 11.3% | 5.1% | 0.9% | 1.0% | 9.8% | 43.2% |
| BB07 | control | 5.3% | 0.0% | 4.4% | 1.8% | 9.9% | 0.0% | 1.0% | 6.8% | 17.2% |
| BB08 | control | 2.1% | 0.0% | 33.7% | 18.7% | 3.9% | 0.0% | 0.0% | 8.2% | 15.9% |
| BB09 | control | 0.9% | 0.0% | 1.4% | 3.1% | 0.0% | 0.0% | 0.0% | 15.4% | 1.2% |
| BB10 | control | 3.5% | 0.0% | 0.9% | 25.9% | 40.3% | 24.0% | 7.8% | 3.8% | 124.0% |

In the table, methylation percentage = the quantitative concentration of a target gene / the quantitative concentration of a reference gene × 100%.

**Table 9 Statistical results**

| | MEIS1 | NKX6-2 | OTX1 | SIM2 | SOX1 | BARHL2 | Nucleic acid fragment 4 | ZNF154 | RUNX3 |
|---|---|---|---|---|---|---|---|---|---|
| Sensitivity | **90.0%** | 60.0% | 70.0% | **90.0%** | 50.0% | 70.0% | **90.0%** | 70.0% | 20.0% |
| Specificity | **90.0%** | 90.0% | 90.0% | **90.0%** | 90.0% | 90.0% | **90.0%** | 90.0% | 90.0% |

The results show that MEIS1, SIM2, and the nucleic acid fragment 4 of the present disclosure can well distinguish the cancer tissue samples from patients with bladder cancer and the bladder tissue samples from people without bladder cancer, and their sensitivity and specificity to the bladder cancer tissue specimens are both 90%.

### Example 4

Sample information: 97 urine samples, including 45 bladder cancer samples and 52 control samples.

In the present example, DNA extraction and transformation were the same as those in Example 3.

Primer and probe sequences are shown in Table 10.

**Table 10**

| Name | Sequence |
|---|---|
| ACTB (reference gene) | Forward primer: TTTTGGATTGTGAATTTGTG (SEQ ID NO: 49) |
| | Reverse primer: AAACCTACTCCTCCCTTAAA (SEQ ID NO: 50) |
| | Probe: TTGTGTGTTGGGTGGTGGTT (SEQ ID NO: 51) |
| Nucleic acid fragment 4 | Forward primer: GCGTTCGGAGTTGTTTAGCG (SEQ ID NO: 72) |
| | Reverse primer: CACCGACGCCACAAACGA (SEQ ID NO: 73) |
| | Probe: CTATTACCGCCGCCGCCGTCG (SEQ ID NO: 74) |
| MEIS1 | Forward primer: GTTCGGGATAAGATTTCGGGG (SEQ ID NO: 75) |
| | Reverse primer: TAATTAAAACTACGCAACCCGACT (SEQ ID NO: 76) |
| | Probe: CGAGAGGGGTCGGGCGAGTTAG (SEQ ID NO: 77) |

An amplification system of the present example is shown in Table 11.

**Table 11**

| Reaction component | Addition amount (µL) |
|---|---|
| 5× buffer | 4 |
| Magnesium ions (25 mM) | 4 |
| dNTPs (10 mM) | 0.8 |
| Forward primer F (25 µM) | 0.4 |
| Reverse primer R (25 µM) | 0.4 |
| Probe P (10 µM) | 0.4 |
| Taq polymerase (5 units/µL) | 0.4 |
| H₂O | 7.6 |
| Template DNA | 2 |
| Total volume | 20 |

An amplification protocol of the present example is shown in Table 12.

**Table 12**

| **step** | **temperature and time** | **number of cycles** |
|---|---|---|
| pre-denaturation | 95°C for 5 min | 1 |
| amplification | 95°C for 20 s | 45 |
| | 65 °C for 20 s | |
| | 62 °C for 40 s | |
| cooling | 40°C for 3 min | 1 |

In the 97 urine samples, if a Ct value of methylation level detection is greater than a cut-off value, the methylation is determined as negative, and otherwise, the methylation is determined as positive. A cut-off value for detection of the nucleic acid fragment 4 is 34.65, and a cut-off value for detection of MEIS1 is 33.82. Detection results are shown in Table 13.

**Table 13 Detection results of the nucleic acid fragment 4 and MEIS 1 in 97 urine samples**

| **Sample no.** | **Sample type** | **Nucleic acid fragment 4** | | **MEIS1** | |
|---|---|---|---|---|---|
| | | **Ct value** | **Detection result** | **Ct value** | **Detection result** |
| UF001 | control | 35.21 | negative | 34.82 | negative |
| UF002 | control | 35.59 | negative | 35.67 | negative |
| UF003 | control | 45 | negative | 36.27 | negative |
| UF004 | control | 45 | negative | 38.84 | negative |
| UF005 | control | 37.64 | negative | 36.16 | negative |
| UF006 | control | 35.74 | negative | 33.77 | ***positive*** |
| UF007 | control | 35.29 | negative | 36.04 | negative |
| UF008 | control | 35.78 | negative | 35.24 | negative |
| UF009 | control | 38.69 | negative | 33.45 | ***positive*** |
| UF010 | control | 36.29 | negative | 35.19 | negative |
| UF011 | control | 45 | negative | 37.9 | negative |
| UF012 | control | 45 | negative | 38.24 | negative |
| UF013 | control | 35.66 | negative | 34.53 | negative |
| UF014 | control | 36.01 | negative | 33.99 | negative |
| UF015 | control | 45 | negative | 37.7 | negative |
| UF016 | control | 35.51 | negative | 43 | negative |
| UF017 | control | 38.13 | negative | 37.21 | negative |
| UF018 | control | 36.81 | negative | 36.47 | negative |
| UF019 | control | 35.47 | negative | 38.45 | negative |
| UF020 | control | 38.94 | negative | 36.76 | negative |
| UA009 | control | 37.26 | negative | 34.56 | negative |
| UA011 | control | 35.81 | negative | 35.53 | negative |
| UA013 | control | 39.42 | negative | 38.74 | negative |
| UA018 | control | 37.11 | negative | 35.1 | negative |
| UA022 | control | 37 | negative | 35.63 | negative |
| UA023 | control | 34.74 | negative | 36.69 | negative |
| UA027 | control | 45 | negative | 35.29 | negative |
| UA028 | control | 36.54 | negative | 33.88 | negative |
| UA029 | control | 38.01 | negative | 34.08 | negative |
| UA030 | control | 37.43 | negative | 35.35 | negative |
| UA032 | control | 37.35 | negative | 35.88 | negative |
| UA037 | control | 45 | negative | 36.94 | negative |
| UA040 | control | 39.99 | negative | 35.97 | negative |
| UA042 | control | 36.98 | negative | 35.32 | negative |
| UA046 | control | 45 | negative | 34.91 | negative |
| UA049 | control | 36.78 | negative | 34.18 | negative |
| UE001 | control | 36.62 | negative | 34.69 | negative |
| UE002 | control | 40.11 | negative | 33.89 | negative |
| UE003 | control | 38.04 | negative | 32.22 | ***positive*** |
| UE004 | control | 35.21 | negative | 32.48 | ***positive*** |
| UE005 | control | 35.85 | negative | 33.81 | ***positive*** |
| UE006 | control | 34.83 | negative | 35.28 | negative |
| UE008 | control | 34.7 | negative | 33.87 | negative |
| UE009 | control | 45 | negative | 35.98 | negative |
| UE010 | control | 36.34 | negative | 35 | negative |
| UE013 | control | 45 | negative | 35.5 | negative |
| UE014 | control | 36.1 | negative | 32.03 | ***positive*** |
| UE016 | control | 37.54 | negative | 34.6 | negative |
| UE017 | control | 37.84 | negative | 31.55 | ***positive*** |
| UE018 | control | 38.09 | negative | 33.71 | ***positive*** |
| UE019 | control | 39.16 | negative | 35.08 | negative |
| UE020 | control | 36.56 | negative | 36.16 | negative |
| UC107 | bladder cancer | 26.19 | positive | 30.05 | positive |
| UC108 | bladder cancer | 27.43 | positive | 31.65 | positive |
| UD043 | bladder cancer | 34.26 | positive | 38.77 | ***negative*** |
| UD044 | bladder cancer | 28.51 | positive | 31.87 | positive |
| UD046 | bladder cancer | 31.21 | positive | 33.72 | positive |
| UD047 | bladder cancer | 28.08 | positive | 32 | positive |
| UD049 | bladder cancer | 34.4 | positive | 35.46 | ***negative*** |
| UD051 | bladder cancer | 27.47 | positive | 31.34 | positive |
| UD054 | bladder cancer | 28.2 | positive | 31.28 | positive |
| UD056 | bladder cancer | 27.87 | positive | 31.01 | positive |
| UD057 | bladder cancer | 26.69 | positive | 30.22 | positive |
| UD059 | bladder cancer | 31.27 | positive | 33.76 | positive |
| UD060 | bladder cancer | 28.24 | positive | 37.69 | ***negative*** |
| UD063 | bladder cancer | 28.89 | positive | 33.08 | positive |
| UD065 | bladder cancer | 30.45 | positive | 33.72 | positive |
| UD068 | bladder cancer | 31.03 | positive | 33.82 | ***negative*** |
| UD070 | bladder cancer | 30.46 | positive | 33.93 | ***negative*** |
| UD072 | bladder cancer | 34.65 | ***negative*** | 34.76 | ***negative*** |
| UD076 | bladder cancer | 27.43 | positive | 31.16 | positive |
| UD084 | bladder cancer | 31.18 | positive | 38.69 | ***negative*** |
| UD089 | bladder cancer | 35.94 | ***negative*** | 31.28 | positive |
| UA001 | bladder cancer | 39.38 | ***negative*** | 37.19 | ***negative*** |
| UA002 | bladder cancer | 34.58 | positive | 33.91 | ***negative*** |
| UA003 | bladder cancer | 30.83 | positive | 29.99 | positive |
| UA004 | bladder cancer | 27.54 | positive | 30.54 | positive |
| UA063 | bladder cancer | 27.48 | positive | 29.67 | positive |
| UA066 | bladder cancer | 27.38 | positive | 30.61 | positive |
| UA067 | bladder cancer | 33.04 | positive | 35.16 | ***negative*** |
| UA068 | bladder cancer | 31.82 | positive | 33.7 | positive |
| UA069 | bladder cancer | 34.33 | positive | 38.53 | ***negative*** |
| UA070 | bladder cancer | 29.89 | positive | 32.33 | positive |
| UA071 | bladder cancer | 25.65 | positive | 28.54 | positive |
| UA072 | bladder cancer | 36.09 | ***negative*** | 27.93 | positive |
| UC018 | bladder cancer | 30.49 | positive | 34.31 | ***negative*** |
| UC035 | bladder cancer | 25.15 | positive | 27.93 | positive |
| UC045 | bladder cancer | 34.14 | positive | 38.61 | ***negative*** |
| UC062 | bladder cancer | 34.52 | positive | 31.73 | positive |
| UC068 | bladder cancer | 33.77 | positive | 33.31 | positive |
| UC070 | bladder cancer | 26.54 | positive | 29.28 | positive |
| UC080 | bladder cancer | 26.07 | positive | 30.74 | positive |
| UC082 | bladder cancer | 38.23 | ***negative*** | 37.59 | ***negative*** |
| UC083 | bladder cancer | 32.52 | positive | 33.67 | positive |
| UC087 | bladder cancer | 29.51 | positive | 32.2 | positive |
| UC092 | bladder cancer | 29.64 | positive | 31.9 | positive |
| UC095 | bladder cancer | 26.63 | positive | 31.88 | positive |

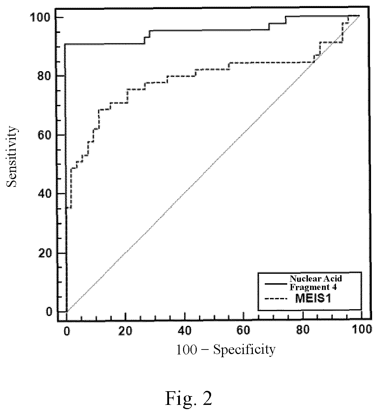

Statistical analysis results of the above detection results are shown in Table 14, and ROC curves are shown in Fig. 2.

**Table 14**

| | Nucleic acid fragment 4 | MEIS1 |
|---|---|---|
| Cut-Off | 34.65 | 33.82 |
| Sensitivity | 91.1 | 71.1 |
| Specificity | 100.0 | 84.6 |
| AUC | 0.956 | 0.790 |

The results show that in detection of methylation levels of the nucleic acid fragment 4 in the urine samples to detect bladder cancer, the detection specificity is as high as 100%, and the sensitivity is 91.1%. In the detection of methylation levels of gene MEIS1 in the urine samples to detect bladder cancer, the detection specificity is 84.6% only, and the sensitivity is 71.1% only.

### Example 5

Sample information: 299 urine samples, including 1 low-malignant-potential inverted urothelial tumor sample, 16 low-malignant-potential papillary urothelial tumor samples, 105 bladder cancer samples, 31 prostate cancer samples, 17 renal pelvis cancer samples, 10 ureteral cancer samples, and 119 control samples.

In the present disclosure, DNA extraction and transformation, primer and probe sequences for the nucleic acid fragment 4, an amplification system, and an amplification protocol were the same as those in Example 4.

Detection results of the nucleic acid fragment 4 in different types of tumor samples are shown in Table 15.

**Table 15 Detection results of the nucleic acid fragment 4 in different types of tumors in urine samples**

| | Sample type | Ct value of the nucleic acid fragment 4 | Detection result of the nucleic acid fragment 4 |
|---|---|---|---|
| UA126 | low-malignant-potential inverted urothelial tumor | 37.89 | negative |
| UD007 | low-malignant-potential papillary urothelial tumor | 45 | negative |
| UD035 | low-malignant-potential papillary urothelial tumor | 39.18 | negative |
| UD055 | low-malignant-potential papillary urothelial tumor | 36.92 | negative |
| UD058 | low-malignant-potential papillary urothelial tumor | 36.77 | negative |
| UD061 | low-malignant-potential papillary urothelial tumor | 31.52 | positive |
| UD091 | low-malignant-potential papillary urothelial tumor | 40.48 | negative |
| UD106 | low-malignant-potential papillary urothelial tumor | 33.11 | positive |
| UD123 | low-malignant-potential papillary urothelial tumor | 37.45 | negative |
| UD159 | low-malignant-potential papillary urothelial tumor | 34.93 | positive |
| UD160 | low-malignant-potential papillary urothelial tumor | 35.19 | positive |
| UD181 | low-malignant-potential papillary urothelial tumor | 38.92 | negative |
| UD230 | low-malignant-potential papillary urothelial tumor | 37.64 | negative |
| UD232 | low-malignant-potential papillary urothelial tumor | 45 | negative |
| UD319 | low-malignant-potential papillary urothelial tumor | 38.82 | negative |
| UG043 | low-malignant-potential papillary urothelial tumor | 36.51 | negative |
| UC071 | low-malignant-potential papillary urothelial tumor | 28.56 | ***positive*** |
| UA010 | control | 37.29 | negative |
| UA012 | control | 45 | negative |
| UA014 | control | 45 | negative |
| UA015 | control | 45 | negative |
| UA016 | control | 36.49 | negative |
| UA017 | control | 37.42 | negative |
| UA020 | control | 36.91 | negative |
| UA021 | control | 45 | negative |
| UA024 | control | 45 | negative |
| UA025 | control | 36.85 | negative |
| UA026 | control | 45 | negative |
| UA031 | control | 45 | negative |
| UA033 | control | 45 | negative |
| UA034 | control | 35.75 | positive |
| UA035 | control | 38.62 | negative |
| UA036 | control | 45 | negative |
| UA039 | control | 45 | negative |
| UA041 | control | 34.56 | ***positive*** |
| UA043 | control | 45 | negative |
| UA044 | control | 37.17 | negative |
| UA045 | control | 37.63 | negative |
| UA047 | control | 33.25 | ***positive*** |
| UA048 | control | 37.92 | negative |
| UA050 | control | 45 | negative |
| UA051 | control | 37.24 | negative |
| UA052 | control | 38.46 | negative |
| UA108 | control | 36.84 | negative |
| UA109 | control | 40.73 | negative |
| UA114 | control | 37.47 | negative |
| UA117 | control | 38.63 | negative |
| UA118 | control | 39.81 | negative |
| UA128 | control | 42.86 | negative |
| UC001 | control | 34.94 | ***positive*** |
| UC002 | control | 36.77 | negative |
| UC005 | control | 37.97 | negative |
| UC006 | control | 45 | negative |
| UC007 | control | 37.7 | negative |
| UC009 | control | 38.15 | negative |
| UC011 | control | 38.43 | negative |
| UC012 | control | 38.92 | negative |
| UC013 | control | 37.17 | negative |
| UC017 | control | 37.49 | negative |
| UC019 | control | 37.67 | negative |
| UC020 | control | 37.79 | negative |
| UC022 | control | 37.67 | negative |
| UC023 | control | 38.4 | negative |
| UC024 | control | 39.64 | negative |
| UC026 | control | 37.81 | negative |
| UC027 | control | 45 | negative |
| UC030 | control | 37.23 | negative |
| UC031 | control | 45 | negative |
| UC032 | control | 36.74 | negative |
| UC033 | control | 38.47 | negative |
| UC039 | control | 37.73 | negative |
| UC040 | control | 38.62 | negative |
| UC041 | control | 37.8 | negative |
| UC044 | control | 38.03 | negative |
| UC046 | control | 34.15 | positive |
| UC047 | control | 37.57 | negative |
| UC048 | control | 36.51 | negative |
| UC051 | control | 45 | negative |
| UC052 | control | 37.56 | negative |
| UC055 | control | 45 | negative |
| UC056 | control | 45 | negative |
| UC059 | control | 45 | negative |
| UC060 | control | 39.64 | negative |
| UC061 | control | 45 | negative |
| UC063 | control | 37.51 | negative |
| UC064 | control | 38.07 | negative |
| UC081 | control | 36.53 | negative |
| UC097 | control | 37.37 | negative |
| UC098 | control | 37.81 | negative |
| UD008 | control | 45 | negative |
| UD040 | control | 39.98 | negative |
| UD041 | control | 35.93 | positive |
| UD045 | control | 37.53 | negative |
| UD050 | control | 38.19 | negative |
| UD071 | control | 38.29 | negative |
| UD073 | control | 37.69 | negative |
| UD108 | control | 37.99 | negative |
| UD124 | control | 39.18 | negative |
| UD126 | control | 37.49 | negative |
| UD200 | control | 42.77 | negative |
| UD236 | control | 45 | negative |
| UD238 | control | 37.68 | negative |
| UD242 | control | 37.06 | negative |
| UD259 | control | 34.49 | ***positive*** |
| UD263 | control | 45 | negative |
| UD265 | control | 45 | negative |
| UD266 | control | 38.52 | negative |
| UD269 | control | 37.42 | negative |
| UD270 | control | 36.66 | negative |
| UD274 | control | 38.54 | negative |
| UD276 | control | 45 | negative |
| UD277 | control | 38.73 | negative |
| UD281 | control | 39.23 | negative |
| UD301 | control | 38.06 | negative |
| UD304 | control | 45 | negative |
| UD306 | control | 36.66 | negative |
| UD316 | control | 38.57 | negative |
| UD318 | control | 36.85 | negative |
| UD323 | control | 39.11 | negative |
| UD325 | control | 38.66 | negative |
| UD326 | control | 38.6 | negative |
| UD328 | control | 40.71 | negative |
| UD331 | control | 37.89 | negative |
| UD336 | control | 32.95 | ***positive*** |
| UG004 | control | 37.95 | negative |
| UG009 | control | 45 | negative |
| UG021 | control | 45 | negative |
| UG036 | control | 38.4 | negative |
| UG039 | control | 45 | negative |
| UG066 | control | 38.08 | negative |
| UG093 | control | 45 | negative |
| UD164 | control | 36.5 | negative |
| UG035 | control | 38.61 | negative |
| UD346 | control | 37.55 | negative |
| UD357 | control | 37.33 | negative |
| UD364 | control | 45 | negative |
| UA002-2 | bladder cancer | 35.86 | positive |
| UA006 | bladder cancer | 33.2 | positive |
| UA008 | bladder cancer | 29.04 | positive |
| UA053 | bladder cancer | 30.97 | positive |
| UA055 | bladder cancer | 29.19 | positive |
| UA056 | bladder cancer | 28.83 | positive |
| UA057 | bladder cancer | 37.26 | ***negative*** |
| UA060 | bladder cancer | 32.61 | positive |
| UA061 | bladder cancer | 37.8 | ***negative*** |
| UA062 | bladder cancer | 29.53 | positive |
| UA073 | bladder cancer | 29.92 | positive |
| UA074 | bladder cancer | 34.94 | positive |
| UA077 | bladder cancer | 32.69 | positive |
| UA078 | bladder cancer | 29.86 | positive |
| UA079 | bladder cancer | 35.04 | positive |
| UA090 | bladder cancer | 36.6 | ***negative*** |
| UA091 | bladder cancer | 29.57 | positive |
| UA092 | bladder cancer | 30.71 | positive |
| UA094 | bladder cancer | 31.85 | positive |
| UA097 | bladder cancer | 29.41 | positive |
| UA100 | bladder cancer | 36.99 | ***negative*** |
| UA104 | bladder cancer | 37.5 | ***negative*** |
| UA111 | bladder cancer | 31.03 | positive |
| UA115 | bladder cancer | 34.47 | positive |
| UA116 | bladder cancer | 31.71 | positive |
| UA119 | bladder cancer | 33.26 | positive |
| UA121 | bladder cancer | 31.05 | positive |
| UA122 | bladder cancer | 30.28 | positive |
| UA124 | bladder cancer | 29.37 | positive |
| UA134 | bladder cancer | 29.32 | positive |
| UA135 | bladder cancer | 36.85 | ***negative*** |
| UA137 | bladder cancer | 31.16 | positive |
| UA138 | bladder cancer | 30.48 | positive |
| UA139 | bladder cancer | 28.75 | positive |
| UA141 | bladder cancer | 28.14 | positive |
| UA147 | bladder cancer | 45 | ***negative*** |
| UA149 | bladder cancer | 29.24 | positive |
| UA154 | bladder cancer | 31.17 | positive |
| UB001 | bladder cancer | 31.57 | positive |
| UB003 | bladder cancer | 29.23 | positive |
| UB005 | bladder cancer | 28.74 | positive |
| UB006 | bladder cancer | 31.12 | positive |
| UB008 | bladder cancer | 32.25 | positive |
| UC096 | bladder cancer | 36.11 | positive |
| UC101 | bladder cancer | 30.2 | positive |
| UD003 | bladder cancer | 35.16 | positive |
| UD004 | bladder cancer | 29.16 | positive |
| UD009 | bladder cancer | 29.79 | positive |
| UD011 | bladder cancer | 28.87 | positive |
| UD014 | bladder cancer | 32.92 | positive |
| UD015 | bladder cancer | 34.54 | positive |
| UD016 | bladder cancer | 30.08 | positive |
| UD018 | bladder cancer | 32.11 | positive |
| UD028 | bladder cancer | 34.16 | positive |
| UD030 | bladder cancer | 28.73 | positive |
| UD038 | bladder cancer | 29.54 | positive |
| UD039 | bladder cancer | 36.31 | negative |
| UD078 | bladder cancer | 33.22 | positive |
| UD080 | bladder cancer | 28.36 | positive |
| UD095 | bladder cancer | 31.15 | positive |
| UD099 | bladder cancer | 35.08 | positive |
| UD109 | bladder cancer | 30.96 | positive |
| UD110 | bladder cancer | 36.1 | positive |
| UD111 | bladder cancer | 32.91 | positive |
| UD134 | bladder cancer | 33.59 | positive |
| UD135 | bladder cancer | 33.21 | positive |
| UD136 | bladder cancer | 28.59 | positive |
| UD137 | bladder cancer | 35.82 | positive |
| UD141 | bladder cancer | 34.57 | positive |
| UD142 | bladder cancer | 34.63 | positive |
| UD145 | bladder cancer | 29.86 | positive |
| UD150 | bladder cancer | 34.84 | positive |
| UD161 | bladder cancer | 30.01 | positive |
| UD162 | bladder cancer | 35.01 | positive |
| UD174 | bladder cancer | 34.83 | positive |
| UD175 | bladder cancer | 32.68 | positive |
| UD205 | bladder cancer | 30.64 | positive |
| UD210 | bladder cancer | 34.01 | positive |
| UD220 | bladder cancer | 29.11 | positive |
| UD221 | bladder cancer | 29.06 | positive |
| UD225 | bladder cancer | 37.17 | ***negative*** |
| UD237 | bladder cancer | 33.03 | positive |
| UD243 | bladder cancer | 36.29 | positive |
| UD244 | bladder cancer | 30.27 | positive |
| UD257 | bladder cancer | 29.87 | positive |
| UD268 | bladder cancer | 29.51 | positive |
| UD293 | bladder cancer | 32.53 | positive |
| UD298 | bladder cancer | 30.48 | positive |
| UD303 | bladder cancer | 32.98 | positive |
| UD308 | bladder cancer | 34.96 | positive |
| UD312 | bladder cancer | 35.02 | positive |
| UD314 | bladder cancer | 31.95 | positive |
| UD330 | bladder cancer | 30.06 | positive |
| UD349 | bladder cancer | 32.5 | positive |
| UD355 | bladder cancer | 30.59 | positive |
| UD361 | bladder cancer | 33.99 | positive |
| UD366 | bladder cancer | 31.61 | positive |
| UD367 | bladder cancer | 31.71 | positive |
| UG003 | bladder cancer | 32.28 | positive |
| UG025 | bladder cancer | 35.58 | positive |
| UG046 | bladder cancer | 34.94 | positive |
| UG053 | bladder cancer | 33.5 | positive |
| UG059 | bladder cancer | 30.67 | positive |
| UG064 | bladder cancer | 32.83 | positive |
| UG080 | bladder cancer | 32 | positive |
| UA088 | prostate cancer | 35.19 | positive |
| UA089 | prostate cancer | 34.53 | positive |
| UA093 | prostate cancer | 36.14 | positive |
| UA105 | prostate cancer | 36.46 | ***negative*** |
| UD064 | prostate cancer | 34.91 | positive |
| UD 102 | prostate cancer | 45 | ***negative*** |
| UD127 | prostate cancer | 36.5 | ***negative*** |
| UD138 | prostate cancer | 37.08 | ***negative*** |
| UD151 | prostate cancer | 36.07 | positive |
| UD154 | prostate cancer | 36.95 | ***negative*** |
| UD165 | prostate cancer | 37.88 | ***negative*** |
| UD179 | prostate cancer | 35.7 | positive |
| UD206 | prostate cancer | 39.03 | ***negative*** |
| UD233 | prostate cancer | 35.56 | positive |
| UD240 | prostate cancer | 34.29 | positive |
| UD254 | prostate cancer | 37.11 | ***negative*** |
| UD255 | prostate cancer | 45 | ***negative*** |
| UD294 | prostate cancer | 45 | ***negative*** |
| UD310 | prostate cancer | 37.97 | ***negative*** |
| UD338 | prostate cancer | 37.18 | ***negative*** |
| UD341 | prostate cancer | 38.13 | ***negative*** |
| UD351 | prostate cancer | 38.71 | ***negative*** |
| UG007 | prostate cancer | 34.69 | positive |
| UG011 | prostate cancer | 37.87 | ***negative*** |
| UG037 | prostate cancer | 38.11 | ***negative*** |
| UG038 | prostate cancer | 45 | ***negative*** |
| UG044 | prostate cancer | 33.04 | positive |
| UG054 | prostate cancer | 36.13 | positive |
| UG061 | prostate cancer | 36.66 | ***negative*** |
| UA110 | prostate cancer | 45 | ***negative*** |
| UD292 | prostate cancer | 45 | ***negative*** |
| UA080 | renal pelvis cancer | 30.98 | positive |
| UA085 | renal pelvis cancer | 34.92 | positive |
| UA086 | renal pelvis cancer | 35.11 | positive |
| UA106 | renal pelvis cancer | 33.1 | positive |
| UA142 | renal pelvis cancer | 37.85 | ***negative*** |
| UD013 | renal pelvis cancer | 35.76 | positive |
| UD023 | renal pelvis cancer | 30.23 | positive |
| UD042 | renal pelvis cancer | 33.77 | positive |
| UD062 | renal pelvis cancer | 35.71 | positive |
| UD067 | renal pelvis cancer | 38.29 | ***negative*** |
| UD079 | renal pelvis cancer | 31.58 | positive |
| UD113 | renal pelvis cancer | 28.25 | positive |
| UD125 | renal pelvis cancer | 30.63 | positive |
| UD251 | renal pelvis cancer | 31.07 | positive |
| UD290 | renal pelvis cancer | 36.56 | ***negative*** |
| UD291 | renal pelvis cancer | 31.54 | positive |
| UD053 | renal pelvis cancer | 31 | positive |
| UD167 | ureteral cancer | 29.78 | positive |
| UD094 | ureteral cancer | 33.57 | positive |
| UD156 | ureteral cancer | 36.74 | ***negative*** |
| UD176 | ureteral cancer | 29.94 | positive |
| UD177 | ureteral cancer | 31.76 | positive |
| UD246 | ureteral cancer | 31.02 | positive |
| UD253 | ureteral cancer | 34.98 | positive |
| UD350 | ureteral cancer | 31.66 | positive |
| UG078 | ureteral cancer | 34.14 | positive |
| UG085 | ureteral cancer | 30.01 | positive |

In the detection, if a Ct value of detection of ACTB is less than 32, it is indicated that the sample is qualified or the operation is correct. If a Ct value of detection of a methylation level of the nucleic acid fragment 4 is less than 36.3, it is indicated that a detection result is positive, and otherwise, the detection result is negative.

Statistical analysis results of the above detection results are shown in Table 16, and ROC curves are shown in Fig. 3.

**Table 16**

| **Analysis group** | **Indicator** | **Nucleic acid fragment 4** |
|---|---|---|
| Comparison of a control group and a bladder cancer & ureteral cancer & renal pelvis cancer group | Specificity | 93.3% |
| | Sensitivity | 90.9% |
| | AUC | 0.962 (P<0.001) |
| Comparison of a control group and a ureteral cancer group | Specificity | 92.4% |
| | Sensitivity | 90.0% |
| | AUC | 0.979 (P<0.001) |
| Comparison of a control group and a renal pelvis cancer group | Specificity | 92.4% |
| | Sensitivity | 82.4% |
| | AUC | 0.927 (P<0.001) |
| Comparison of a control group and a bladder cancer group | Specificity | 93.3% |
| | Sensitivity | 90.4% |
| | AUC | 0.966 (P<0.001) |
| Comparison of a control group and | Specificity | 92.4 |
| a low-malignant-potential inverted urothelial tumor & low-malignant-potential papillary urothelial tumor group | Sensitivity | 83.6 |
| | AUC | 0.923 (P<0.001) |
| Comparison of a control group and a prostate cancer group | Specificity | 80.7 |
| | Sensitivity | 58.1 |
| | AUC | 0.671 (P=0.006) |

It can be seen from the results in the above tables, the nucleic acid fragment 4 has high sensitivity and specificity for detection of various types of tumors in the urine samples.

### Example 6

Primers and probes also have a great influence on detection effects of tumor markers. In the course of the research, the inventors designed multiple pairs of primers and their corresponding probes to screen out probes and primers that can improve the detection sensitivity and specificity as much as possible in order to enable the detection reagent of the present disclosure to be practically applied to clinical detection. Some primers and probes (6 sets) are shown in Table 17, and detection results are shown in Table 18. All the primers and probes were designed by the inventors using a methylated sequence of the nucleic acid fragment 4 that was obtained by transformation with a sulfite as a template. They were synthesized by Sangon Biotech (Shanghai) Co., Ltd.

**Table 17 Primers and probes**

| **Name** | **Sequence No.** | **Sequence** |
|---|---|---|
| CG443-F | SEQ ID NO.: 31 | AGGTTCGTTTACGAGGTTTTC |
| CG443-R | SEQ ID NO.: 32 | CCTACGCCAACTACTCCG |
| CG443-P | SEQ ID NO.: 33 | CGAACGCTCCCGCTCCAAA |
| CG447-F | SEQ ID NO.: 34 | TGCGTTAAGTGTACGTTTATC |
| CG447-R | SEQ ID NO.: 35 | CGTAAAACAACTACAACTCGCG |
| CG447-P | SEQ ID NO.: 36 | TTCTCCTCCTACGCCTACTACCTA |
| CG190-F | SEQ ID NO.: 37 | GCGTTGCGTAGGTAGTAGGC |
| CG190-R | SEQ ID NO.: 38 | GAACCTCGAAAAAATAATACCGTT |
| CG190-P | SEQ ID NO.: 39 | AGGAGAACGAGGCGCGCGA |
| CG437-F | SEQ ID NO.: 40 | GCGTTCGGAGTTGTTTAGCG |
| CG437-R | SEQ ID NO.: 41 | CACCGACGCCACAAACGA |
| CG437-P | SEQ ID NO.: 42 | CTATTACCGCCGCCGCCGTCG |
| CG441-F | SEQ ID NO.: 43 | GCGGTCGTTGTATCGTTATC |
| CG441-R | SEQ ID NO.: 44 | GAATACTTCTCGACTACCCG |
| CG441-P | SEQ ID NO.: 45 | TAACGACCCCCGCAACAAACCG |
| CG446-F | SEQ ID NO.: 46 | CGTTGTATCGTTATCGGTGAGC |
| CG446-R | SEQ ID NO.: 47 | GCGCACTTAAAAATCCGCG |
| CG446-P | SEQ ID NO.: 48 | CTTCTCGACTACCCGCAACAACAATAACG |
| A1-F | SEQ ID NO.: 78 | TTGGATTGTGAATTTGTGTTTGT |
| A1-R | SEQ ID NO.: 79 | CAATAAAACCTACTCCTCCCTTA |
| A1-P | SEQ ID NO.: 51 | TTGTGTGTTGGGTGGTGGTT |
| A2-F | SEQ ID NO.: 80 | GATGGAGGAGGTTTAGTAAGTT |
| A2-R | SEQ ID NO.: 81 | CAATAAAACCTACTCCTCCCTTA |
| A2-P | SEQ ID NO.: 51 | TTGTGTGTTGGGTGGTGGTT |
| A3-F | SEQ ID NO.: 82 | GGAGGTTTAGTAAGTTTTTTGGATT |
| A3-R | SEQ ID NO.: 83 | CAATAAAACCTACTCCTCCCTTA |
| A3-P | SEQ ID NO.: 51 | TTGTGTGTTGGGTGGTGGTT |

Screening of the primer and probe sets was performed using a 5637 bladder cancer cell line (positive) and an SK-N-BE cell line (negative). By mycoplasma detection and cell morphology analysis, it was determined that the cells were normal. The cells were expanded and collected, and DNA was extracted by using a DNA extraction kit (QIAGEN DNA Mini Kit, #51306) purchased from QIAGEN. The extracted DNA was quantified by using a UV spectrophotometer and then modified with a sulfite by using a DNA transformation kit (EZ DNA Methylation Kit, D5002) purchased from ZYMO RESEARCH. The DNA was diluted with a 1× TE buffer to 6,000 copies/µL. A positive reference P0 (100% positive DNA, that is, the degree of methylation of the nucleic acid fragment 4 was 100%) and a negative reference N0 (100% negative DNA, that is, the degree of methylation of the nucleic acid fragment 4 was 0) were respectively obtained. P1 was obtained by mixing positive DNA with negative DNA in a ratio of 1:9, P2 was obtained by mixing positive DNA with negative DNA in a ratio of 1:99, and P3 was obtained by mixing positive DNA with negative DNA in a ratio of 1:999.

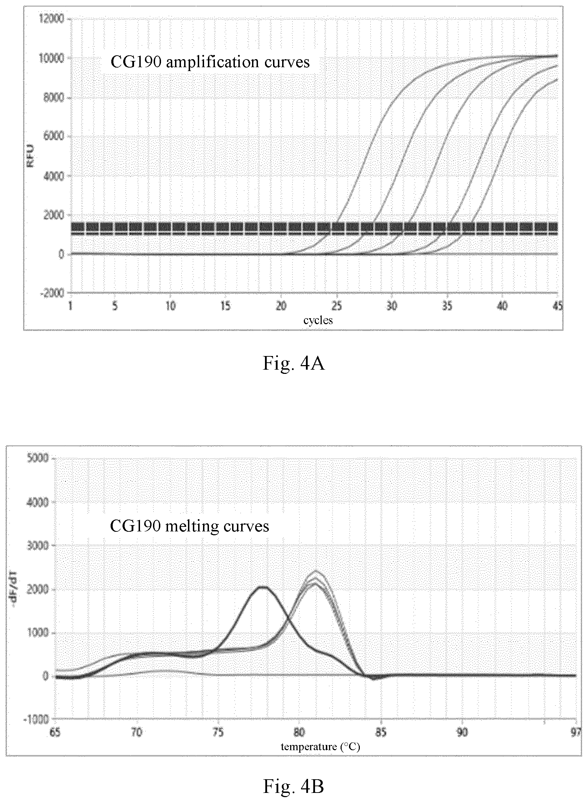

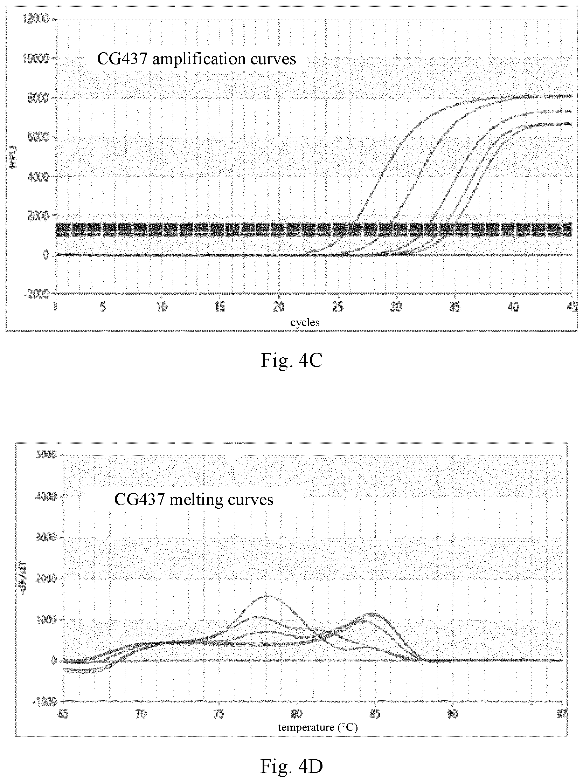

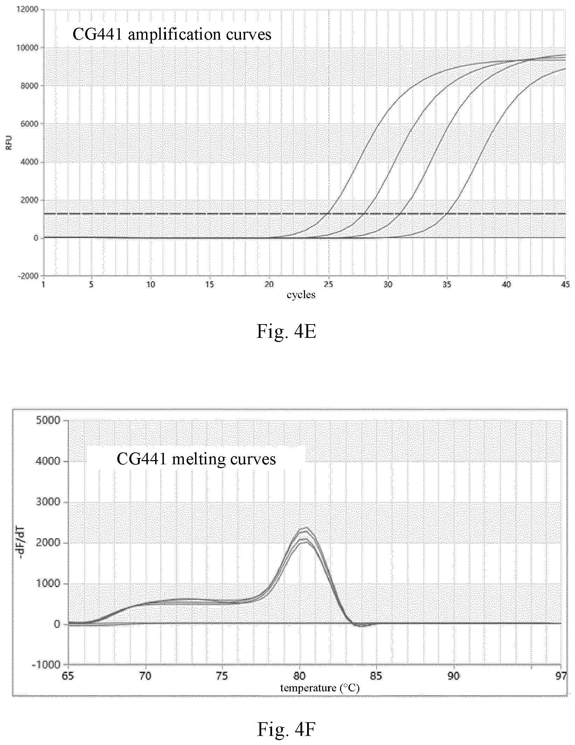

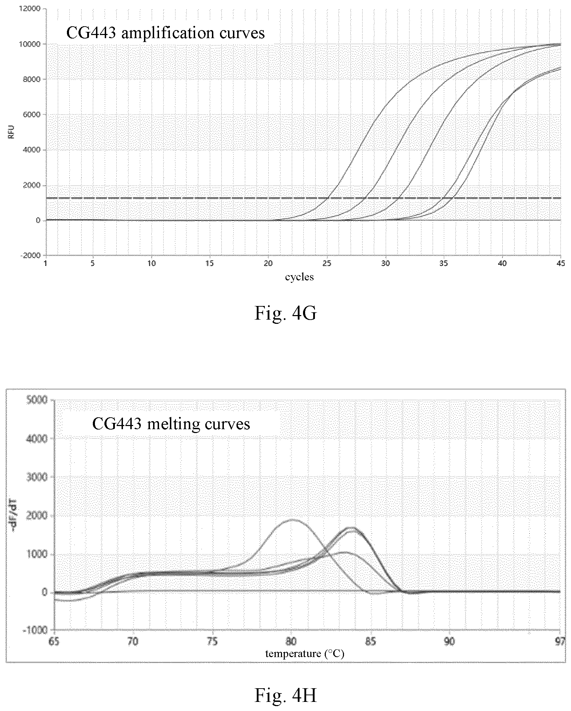

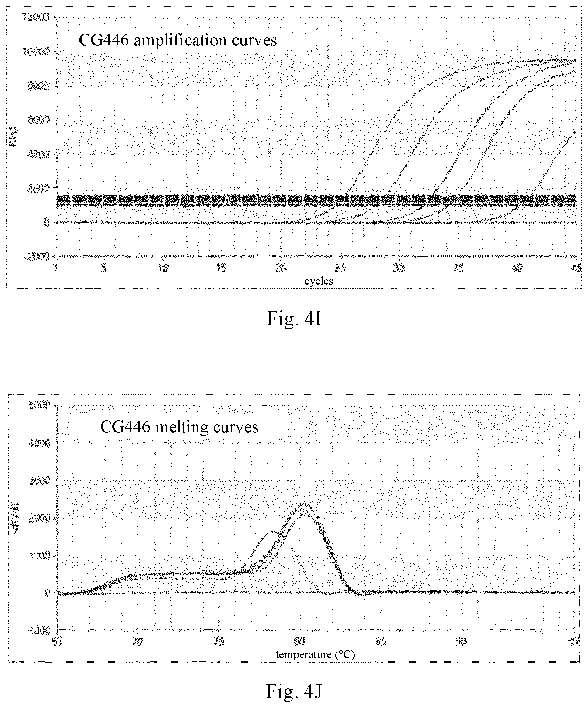

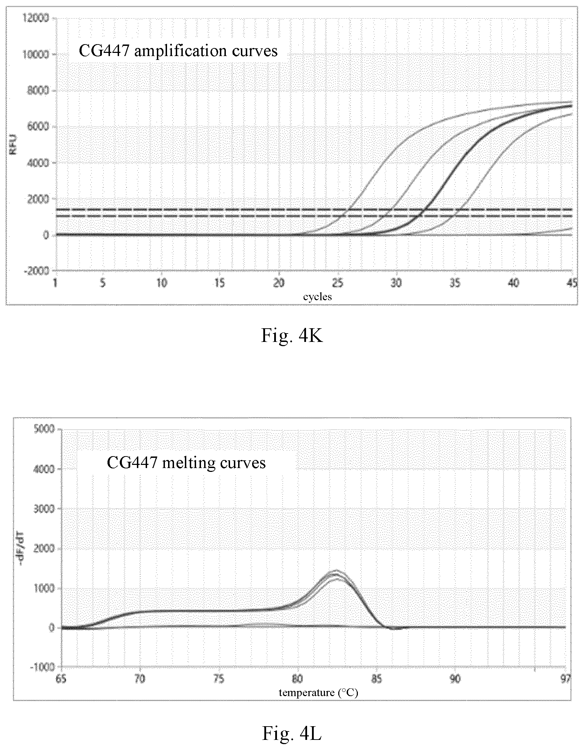

Primer melting curves were obtained according to the system and the amplification protocol of Example 1, and further, amplification results of the primer and probe sets were obtained according to the detection system and the amplification protocol of Example 4. Amplification curves and melting curves of the primer and probe sets are shown in Fig. 4.

Detection result data of the primer and probe sets in different references is shown in Table 18.

**Table 18 Ct values of detection of primer and probe sets in different references**

| Detection of DNA | CG190 | CG437 | CG441 | CG443 | CG446 | CG447 | A1 (internal reference) | A2 (internal reference) | A3 (internal reference) |
|---|---|---|---|---|---|---|---|---|---|
| P0 (LOD 100%) | 24.973 | 25.809 | 24.934 | 25.090 | 25.238 | 25.355 | 24.582 | 24.363 | 24.371 |
| P1 (LOD 10%) | 28.340 | 29.020 | 28.035 | 28.285 | 28.637 | 28.949 | 24.691 | 24.270 | 24.715 |
| P2 (LOD 1%) | 31.520 | 32.309 | 31.090 | 31.145 | 32.637 | 31.902 | 24.504 | 24.285 | 24.559 |
| P3 (LOD 0.1%) | 35.293 | 33.738 | 35.012 | 34.949 | 34.738 | 34.824 | 24.449 | 24.230 | 24.566 |
| N0 (negative) | 37.176 | 34.520 | No amplification | 35.754 | *40.895* | No amplification | 24.793 | 24.449 | 24.840 |

A cut-off value for result determination is 38, if a Ct value is less than or equal to 38, it is indicated that a detection result is positive, and if the Ct value is greater than 38, it is indicated that the detection result is negative.

As shown in Table 18, all the 6 primer and probe combinations can detect the 5637 positive bladder cancer cell line at the limit of detection (LOD) of 0.1%, and only the primer and probe sets CG441, CG446, and CG447 do not cause nonspecific amplification in the SK-N-BE negative cell line or misjudge the cell line as positive. For practical application in clinical, the inventors designed and selected three sets of primer and probe sequences for detecting the reference gene ACTB, the detection and assessment results of the references show that detection results of the primer and probe detection systems A1, A2, and A3 are consistent.

Fig. 4 shows that melting curves of the detection systems CG190, CG437, and CG443 have two peaks, positions of melting peaks in the detection results of the references P0, P1, and P2 are consistent, positions of melting peaks of some detection systems in the detection results of the references P3 and N0 greatly differ from that in the detection result of P0, Ct values of the quantified amplification curves indicate that the detection systems cannot well distinguish the negative reference from the reference P3, and the limit of detection cannot reach one thousandth. A melting curve of the detection system CG446 also has two peaks, a position of a melting peak of CG446 in the detection result of N0 greatly differs from that in the detection result of P0, a Ct value of the quantified amplification curve of CG446 indicates that the detection system can well distinguish the negative reference from the reference P3, and the limit of detection can reach one thousandth. Melting curves of the detection systems CG441 and CG447 have a single peak, there is no amplification curve and no product melting peak in the detection result of N0, Ct values of the quantified amplification curves of CG441 and CG447 indicate that the detection systems can well distinguish the negative reference from the reference P3, and the limit of detection reaches one thousandth. The fluorescence signal intensity of CG447 in the platform stage is significantly lower than those of CG441 and CG446.

### Example 7

The primer and probe set CG441 was detected in 193 urine specimens (including 89 bladder cancer specimens and 104 contrast specimens), and an amplification system and an amplification protocol were the same as those in Example 4. Detection results are shown in Table 19.

**Table 19 Detection results of the primer and probe set CG441 in 193 urine samples**

| Sample No. | Sample type | ACTB (reference gene) | CG441 | Detection result |
|---|---|---|---|---|
| UA141 | bladder cancer | 25.77 | 28.79 | positive |
| UD080 | bladder cancer | 27.98 | 28.55 | positive |
| UD136 | bladder cancer | 28.61 | 28.55 | positive |
| UD030 | bladder cancer | 28.62 | 28.97 | positive |
| UB005 | bladder cancer | 28.09 | 29.01 | positive |
| UA139 | bladder cancer | 27.84 | 29.53 | positive |
| UA056 | bladder cancer | 27.94 | 30.66 | positive |
| UD011 | bladder cancer | 27.88 | 28.89 | positive |
| UA008 | bladder cancer | 28.02 | 31.41 | positive |
| UD221 | bladder cancer | 27.97 | 29.18 | positive |
| UD220 | bladder cancer | 28.07 | 29.25 | positive |
| UD004 | bladder cancer | 28.18 | 29.54 | positive |
| UA055 | bladder cancer | 28.58 | 31.55 | positive |
| UB003 | bladder cancer | 28.53 | 29.88 | positive |
| UA149 | bladder cancer | 27.98 | 29.49 | positive |
| UA134 | bladder cancer | 28.73 | 32.23 | positive |
| UA124 | bladder cancer | 27.25 | 32.72 | positive |
| UA097 | bladder cancer | 28.43 | 30.24 | positive |
| UD268 | bladder cancer | 28.16 | 29.72 | positive |
| UA062 | bladder cancer | 27.8 | 30.46 | positive |
| UD038 | bladder cancer | 28.16 | 30.23 | positive |
| UA091 | bladder cancer | 29.07 | 33.89 | positive |
| UD009 | bladder cancer | 28.09 | 30.74 | positive |
| UD145 | bladder cancer | 28.26 | 29.84 | positive |
| UA078 | bladder cancer | 27.87 | 31.69 | positive |
| UD257 | bladder cancer | 28.27 | 30.65 | positive |
| UA073 | bladder cancer | 28.04 | 31.23 | positive |
| UD161 | bladder cancer | 28.58 | 30.16 | positive |
| UD016 | bladder cancer | 29.27 | 30.28 | positive |
| UC101 | bladder cancer | 28.5 | 30.95 | positive |
| UD244 | bladder cancer | 27.84 | 30.99 | positive |
| UA122 | bladder cancer | 28.31 | 31.12 | positive |
| UD298 | bladder cancer | 28.52 | 30.43 | positive |
| UA138 | bladder cancer | 28.26 | 31.49 | positive |
| UD205 | bladder cancer | 28.63 | 31.62 | positive |
| UA092 | bladder cancer | 28.84 | 31.46 | positive |
| UD 109 | bladder cancer | 28.69 | 31.26 | positive |
| UA053 | bladder cancer | 27.98 | 32.22 | positive |
| UA111 | bladder cancer | 28.61 | 31.82 | positive |
| UA121 | bladder cancer | 28.76 | 31.62 | positive |
| UB006 | bladder cancer | 28.5 | 31.92 | positive |
| UD095 | bladder cancer | 28.02 | 31.17 | positive |
| UA137 | bladder cancer | 31.55 | 31.86 | positive |
| UA154 | bladder cancer | 28.33 | 33.49 | positive |
| UB001 | bladder cancer | 28.79 | 31.74 | positive |
| UA116 | bladder cancer | 28.36 | 32.16 | positive |
| UA094 | bladder cancer | 30.78 | 33.13 | positive |
| UD018 | bladder cancer | 30.32 | 33.01 | positive |
| UB008 | bladder cancer | 28.58 | 32.81 | positive |
| UD293 | bladder cancer | 28.53 | 32.7 | positive |
| UA060 | bladder cancer | 28.7 | 36.1 | positive |
| UD175 | bladder cancer | 28.82 | 33.06 | positive |
| UA077 | bladder cancer | 28.14 | 33.82 | positive |
| UD111 | bladder cancer | 32.89 | 33.47 | positive |
| UD014 | bladder cancer | 28.35 | 32.94 | positive |
| UD336 | control | 29.06 | 39.21 | negative |
| UD303 | bladder cancer | 28.76 | 36.95 | positive |
| UD237 | bladder cancer | 30.92 | 34.63 | positive |
| UA006 | bladder cancer | 28.13 | 40.73 | ***negative*** |
| UD135 | bladder cancer | 28.59 | 33.82 | positive |
| UD078 | bladder cancer | 28.63 | 33.74 | positive |
| UA047 | control | 28.44 | 33.44 | ***positive*** |
| UA119 | bladder cancer | 28.85 | 34.58 | positive |
| UD134 | bladder cancer | 28.85 | 34.18 | positive |
| UD210 | bladder cancer | | 34.47 | positive |
| UC046 | control | 28.98 | 35.98 | ***positive*** |
| UD028 | bladder cancer | 28.32 | 34.14 | positive |
| UA115 | bladder cancer | 30.82 | 35.14 | positive |
| UD259 | control | 28.64 | 37.1 | ***positive*** |
| UD015 | bladder cancer | 28.21 | 36.08 | positive |
| UA041 | control | 28.9 | 38.6 | negative |
| UD141 | bladder cancer | 29.8 | 34.97 | positive |
| UD142 | bladder cancer | 29.92 | 35.47 | positive |
| UD174 | bladder cancer | 29.03 | 36.32 | positive |
| UD150 | bladder cancer | 29.44 | 36.45 | positive |
| UA074 | bladder cancer | 32.62 | 36.47 | positive |
| UC001 | control | 28.89 | 36.78 | ***positive*** |
| UD308 | bladder cancer | 28.52 | 35.41 | positive |
| UA079 | bladder cancer | 29.33 | 40.29 | ***negative*** |
| UD099 | bladder cancer | 28.14 | 36.62 | positive |
| UD003 | bladder cancer | 28.46 | 40.21 | ***negative*** |
| UA034 | control | 28.95 | 39.11 | negative |
| UD137 | bladder cancer | 30.71 | 39.03 | ***negative*** |
| UA002-2 | bladder cancer | 29.72 | 37.66 | positive |
| UD041 | control | 28.7 | 38.65 | negative |
| UD110 | bladder cancer | 28.6 | 37.43 | positive |
| UC096 | bladder cancer | 30.93 | 44 | ***negative*** |
| UD243 | bladder cancer | 31.46 | 36.57 | positive |
| UD039 | bladder cancer | 28.43 | 39.76 | ***negative*** |
| UA016 | control | 28.94 | 43 | negative |
| UD164 | control | 28.73 | 40.47 | negative |
| UC048 | control | 28.21 | 44 | negative |
| UC081 | control | 27.9 | 40.39 | negative |
| UA090 | bladder cancer | 28.71 | 40.83 | ***negative*** |
| UD270 | control | 29.85 | 37.27 | ***positive*** |
| UD306 | control | 28.91 | 44 | negative |
| UC032 | control | 28.93 | 38.9 | negative |
| UC002 | control | 28.58 | 37.82 | ***positive*** |
| UA108 | control | 28.66 | 38.81 | negative |
| UD318 | control | 29.83 | 39.14 | negative |
| UA025 | control | 28.91 | 43 | negative |
| UA135 | bladder cancer | 28.79 | 39.61 | ***negative*** |
| UA020 | control | 28.82 | 43 | negative |
| UA100 | bladder cancer | 28.14 | 44 | ***negative*** |
| UD242 | control | 29.58 | 44 | negative |
| UA044 | control | 29.17 | 44 | negative |
| UC013 | control | 28.33 | 44 | negative |
| UD225 | bladder cancer | 30.63 | 39.58 | ***negative*** |
| UC030 | control | 29.2 | 44 | negative |
| UA051 | control | 28.72 | 39.49 | negative |
| UA057 | bladder cancer | 30.11 | 44 | ***negative*** |
| UA010 | control | 28.93 | 39.99 | negative |
| UD357 | control | 27.73 | 44 | negative |
| UC097 | control | 28.88 | 44 | negative |
| UD269 | control | 30.34 | 40.72 | negative |
| UA017 | control | 29.56 | 44 | negative |
| UA114 | control | 28.04 | 43 | negative |
| UD126 | control | 28.81 | 38.62 | negative |
| UC017 | control | 28.92 | 44 | negative |
| UA104 | bladder cancer | 28.79 | 44 | ***negative*** |
| UC063 | control | 28.27 | 44 | negative |
| UD045 | control | 28.29 | 44 | negative |
| UD346 | control | 29.17 | 44 | negative |
| UC052 | control | 28.07 | 44 | negative |
| UC047 | control | 29.03 | 44 | negative |
| UA045 | control | 28.45 | 44 | negative |
| UC022 | control | 28.93 | 38.73 | negative |
| UC019 | control | 28.94 | 39.08 | negative |
| UD238 | control | 28.2 | 39.73 | negative |
| UD073 | control | 28.31 | 40.61 | negative |
| UC007 | control | 28.61 | 44 | negative |
| UC039 | control | 28.04 | 41.53 | negative |
| UC020 | control | 28.66 | 44 | negative |
| UC041 | control | 28.16 | 44 | negative |
| UA061 | bladder cancer | 28.57 | 39.92 | ***negative*** |
| UC026 | control | 29.23 | 39.74 | negative |
| UC098 | control | 28.68 | 43 | negative |
| UA048 | control | 29.52 | 44 | negative |
| UC005 | control | 29.43 | 40.93 | negative |
| UD108 | control | 28.36 | 44 | negative |
| UC044 | control | 28.83 | 39.09 | negative |
| UC064 | control | 28.87 | 39.75 | negative |
| UC009 | control | 28 | 44 | negative |
| UD050 | control | 28.79 | 44 | negative |
| UD071 | control | 29.67 | 44 | negative |
| UC023 | control | 29.51 | 44 | negative |
| UC011 | control | 28.65 | 44 | negative |
| UA052 | control | 29.08 | 39.13 | negative |
| UC033 | control | 28.84 | 44 | negative |
| UD266 | control | 29.92 | 39.91 | negative |
| UD274 | control | 28.81 | 44 | negative |
| UC040 | control | 29.21 | 40.05 | negative |
| UA035 | control | 31.25 | 44 | negative |
| UA117 | control | 29.24 | 44 | negative |
| UD277 | control | 28.64 | 44 | negative |
| UC012 | control | 28.89 | 44 | negative |
| UD124 | control | 30.65 | 39.12 | negative |
| UD281 | control | 28.62 | 44 | negative |
| UC024 | control | 29.61 | 44 | negative |
| UC060 | control | 31.53 | 44 | negative |
| UA118 | control | 33.15 | 44 | negative |
| UD040 | control | 28.75 | 44 | negative |
| UA109 | control | 31.61 | 44 | negative |
| UD200 | control | 32.69 | 44 | negative |
| UA128 | control | 31.09 | 44 | negative |
| UA147 | bladder cancer | 30.53 | 44 | ***negative*** |
| UA021 | control | 28.93 | 39.63 | negative |
| UD364 | control | 30.84 | 39.72 | negative |
| UC061 | control | 31.94 | 40.68 | negative |
| UA050 | control | 28.88 | 43 | negative |
| UC059 | control | 30.63 | 43 | negative |
| UD263 | control | 30.59 | 43 | negative |
| UD008 | control | 30.29 | 44 | negative |
| UC056 | control | 32.65 | 44 | negative |
| UC055 | control | 28.32 | 44 | negative |
| UC027 | control | 32.18 | 44 | negative |
| UD265 | control | 30.04 | 44 | negative |
| UD236 | control | 28.35 | 44 | negative |
| UA015 | control | 29.47 | 44 | negative |
| UG093 | control | 29.74 | 44 | negative |
| UA014 | control | 31.76 | 44 | negative |
| UA012 | control | 31.47 | 44 | negative |
| UA024 | control | 32.56 | 44 | negative |
| UA036 | control | 30.78 | 44 | negative |
| UA043 | control | 30.74 | 44 | negative |
| UA033 | control | 29.55 | 44 | negative |
| UA031 | control | 29.48 | 44 | negative |
| UA026 | control | 28.84 | 44 | negative |
| UA039 | control | 33 | 44 | negative |
| UC006 | control | 31.89 | 44 | negative |
| UC031 | control | 30.49 | 44 | negative |
| UC051 | control | 31.51 | 44 | negative |
| UD276 | control | 32.49 | 44 | negative |

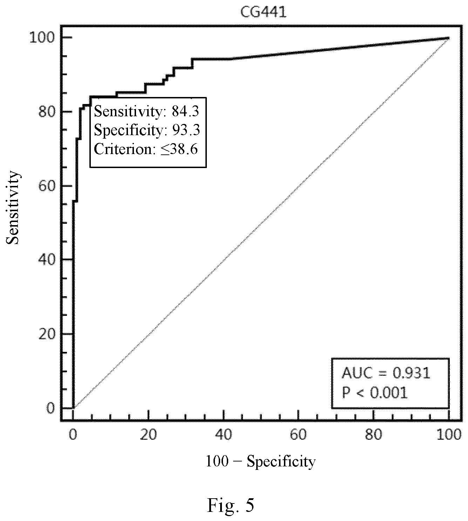

A cut-off value for result determination is set as 38.6, if a Ct value is less than or equal to 38.6, a detection is determined as positive, and if the Ct value is greater than 38.5, the detection result is determined as negative. The obtained detection specificity of the nucleic acid fragment 4 to the bladder cancer samples in the 193 urine samples is 93.3%, the sensitivity is 84.3%, and the area under an ROC curve is 0.931 (P<0.001). The ROC curve is shown in Fig. 5.

### References:

1. Mbeutcha, A., Lucca, I., Mathieu, R., Lotan, Y & Shariat, S. F. Current Status of Urinary Biomarkers for Detection and Surveillance of Bladder Cancer. Urologic Clinics of North America 43, 47-62 (2016).

## Claims

1. A primer, comprising:
a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 31, SEQ ID NO: 32, SEQ ID NO: 34, SEQ ID NO: 35, SEQ ID NO: 37, SEQ ID NO: 38, SEQ ID NO: 40, SEQ ID NO: 41, SEQ ID NO: 43, SEQ ID NO: 44, SEQ ID NO: 46, or SEQ ID NO: 47, or complementary sequences thereof;
optionally, the primer comprising multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of any primer pair shown as SEQ ID NO: 31 and SEQ ID NO: 32, SEQ ID NO: 34 and SEQ ID NO: 35, SEQ ID NO: 37 and SEQ ID NO: 38, SEQ ID NO: 40 and SEQ ID NO: 41, SEQ ID NO: 43 and SEQ ID NO: 44, or SEQ ID NO: 46 and SEQ ID NO: 47;
optionally, the primer comprising multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of any primer pair shown as SEQ ID NO: 34 and SEQ ID NO: 35, SEQ ID NO: 43 and SEQ ID NO: 44, or SEQ ID NO: 46 and SEQ ID NO: 47;
optionally, the primer comprising multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of a primer pair shown as SEQ ID NO: 43 and SEQ ID NO: 44.

2. A probe, comprising:
a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 33, SEQ ID NO: 36, SEQ ID NO: 39, SEQ ID NO: 42, SEQ ID NO: 45, or SEQ ID NO: 48, or complementary sequences thereof;
optionally, the probe comprising a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 36, SEQ ID NO: 45, or SEQ ID NO: 48, or complementary sequences thereof;
optionally, the probe comprising a nucleotide sequence that has at least 85% or at least 90% or at least 91% or at least 92% or at least 93% or at least 94% or at least 95% or at least 96% or at least 97% or at least 98% or at least 99% or 100% identity with a sequence shown as SEQ ID NO: 45.

3. The primer according to claim 1 and/or the probe according to claim 2, for use to detect tumors.

4. Use of the primer according to claim 1 and/or the probe according to claim 2 in preparation of a tumor detection reagent or a kit.

5. Use of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in preparation of a tumor detection reagent or a kit.

6. Use of a reagent for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in preparation of a tumor detection reagent or a kit.

7. A method for detecting the methylation of a nucleotide sequence in a sample, comprising:
treating a sample to be detected with a hydrosulfite or a bisulfite or a hydrazine salt to obtain a modified sample to be detected; and
using a reagent for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 to detect a methylation level of the nucleotide sequence in the modified sample to be detected; optionally, using real-time fluorescence quantitative methylation-specific polymerase chain reaction for detection.

8. A method for detecting a tumor in a subject, comprising:
detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in a sample to be detected from a subject; optionally, a methylation level of the nucleotide sequence in the sample being detected by the detection method according to claim 7; and
indicating whether the subject has or is at risk of having a tumor according to the deviation, optionally, a relative difference, of the methylation level of the nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in the sample from the subject that is obtained by the methylation level detection method, optionally, the methylation level of the nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in the sample from the subject that is detected by the detection method according to claim 7, from a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in a normal control sample.

9. A method for treating a tumor in a subject, comprising:
detecting a tumor in a subject, comprising: detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 in a sample to be detected from the subject; and
treating the tumor in case that the detection of the tumor in the subject indicates that the subject has or is at risk of having the tumor;
optionally, the methylation level of the nucleotide sequence in the sample is detected by the detection method according to claim 7;
optionally, the tumor in the subject is detected by the detection method according to claim 8;
optionally, the treatment is selected from one or more of surgery, chemotherapy, radiotherapy, chemoradiotherapy, immunotherapy, oncolytic virus therapy, and a combination thereof.

10. A tumor detection reagent, comprising a reagent for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4.

11. A kit, comprising the tumor detection reagent according to claim 9.

12. The method, the tumor detection reagent, the kit or the use according to any one of claims 6 to 11, wherein the methylation detection reagent comprises primers and/or a probe for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4;
optionally, a forward primer of the primers has any one of the following nucleotide sequences:
I. nucleotide sequences that have at least 85% or at least 90% or at least 91% or at least 92% or at least 93% or at least 94% or at least 95% or at least 96% or at least 97% or at least 98% or at least 99% or 100% identity with nucleotide sequences shown as SEQ ID NO: 31, SEQ ID NO: 34, SEQ ID NO: 37, SEQ ID NO: 40, SEQ ID NO: 43, or SEQ ID NO: 46, or
II. complementary sequences of the sequences shown in I;
optionally, a reverse primer of the primers has any one of the following nucleotide sequences:
III. nucleotide sequences that have at least 85% or at least 90% or at least 91% or at least 92% or at least 93% or at least 94% or at least 95% or at least 96% or at least 97% or at least 98% or at least 99% or 100% identity with nucleotide sequences shown as SEQ ID NO: 32, SEQ ID NO: 35, SEQ ID NO: 38, SEQ ID NO: 41, SEQ ID NO: 44, or SEQ ID NO: 47, or
IV complementary sequences of the sequences shown in III;
optionally, the primers comprise multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of any primer pair shown as SEQ ID NO: 31 and SEQ ID NO: 32, SEQ ID NO: 34 and SEQ ID NO: 35, SEQ ID NO: 37 and SEQ ID NO: 38, SEQ ID NO: 40 and SEQ ID NO: 41, SEQ ID NO: 43 and SEQ ID NO: 44, or SEQ ID NO: 46 and SEQ ID NO: 47;
optionally, the primers comprise multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of any primer pair shown as SEQ ID NO: 34 and SEQ ID NO: 35, SEQ ID NO: 43 and SEQ ID NO: 44, or SEQ ID NO: 46 and SEQ ID NO: 47;
optionally, the primers comprise multiple nucleotide sequences that respectively have at least 85% or at least 90% or at least 91% or at least 92% or at least 93% or at least 94% or at least 95% or at least 96% or at least 97% or at least 98% or at least 99% or 100% identity with each primer of a primer pair shown as SEQ ID NO: 43 and SEQ ID NO: 44;
optionally, the probe has any one of the following nucleotide sequences:
V. nucleotide sequences that have at least 85% or at least 90% or at least 91% or at least 92% or at least 93% or at least 94% or at least 95% or at least 96% or at least 97% or at least 98% or at least 99% or 100% identity with sequences shown as SEQ ID NO: 33, SEQ ID NO: 36, SEQ ID NO: 39, SEQ ID NO: 42, SEQ ID NO: 45, or SEQ ID NO: 48; or
VI. complementary sequences of the sequences shown in V;
optionally, the probe comprises a nucleotide sequence that has at least 85% or at least 90% or at least 91% or at least 92% or at least 93% or at least 94% or at least 95% or at least 96% or at least 97% or at least 98% or at least 99% or 100% identity with any one of sequences shown as SEQ ID NO: 36, SEQ ID NO: 45, or SEQ ID NO: 48, or complementary sequences thereof;
optionally, the probe comprises a nucleotide sequence that has at least 85% or at least 90% or at least 91% or at least 92% or at least 93% or at least 94% or at least 95% or at least 96% or at least 97% or at least 98% or at least 99% or 100% identity with a sequence shown as SEQ ID NO: 45.

13. The use, the method, the tumor detection reagent or the kit according to any one of claims 3 to 12, wherein the sample to be detected is selected from tissue, body fluid, and excrement;
optionally, the tissue is selected from bladder tissue;
optionally, the body fluid is selected from at least one of blood, serum, plasma, extracellular fluid, tissue fluid, lymph fluid, cerebrospinal fluid, and aqueous humor;
optionally, the excrement is selected from at least one of sputum, urine, saliva, and faeces;
optionally, the excrement is selected from urine.

14. A method for designing primers, comprising steps of designing primers for a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4.

15. A system for designing primers, comprising:
1) an input component;
2) a processing component; and
3) an output component;
optionally, the input component being configured to read a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4;
optionally, the processing component being loaded with a program for designing primers based on information read by the input component;
optionally, the output component be configured to output primer sequences designed by the processing component.

16. A tumor detection system, comprising:
a. a component for detecting a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4; and
b. a result determination system;
optionally, the methylation level detection component comprising the detection reagent or the kit according to any one of claims 10 to 12;
optionally, the result determination component being configured to output the risk of having a tumor and/or a tumor type according to a methylation level of a nucleotide sequence that has at least 85% or at least 90% or at least 91% or at least 92% or at least 93% or at least 94% or at least 95% or at least 96% or at least 97% or at least 98% or at least 99% or 100% identity with a nucleotide sequence shown as SEQ ID NO: 4 that is detected by the detection system;
optionally, the risk of having a tumor being determined by comparing a methylation level of a sample to be detected to a methylation level of a normal sample, and determining that the sample to be detected has a high risk of having a tumor if there is a significant difference or an extremely significant difference between the methylation level of the sample to be detected and the methylation level of the normal sample.

17. The primer, the probe, the use, the nucleotide sequence, the tumor detection reagent, the kit, the method or the system according to any one of claims 3 to 13, and 16, wherein the tumor is selected from urothelial tumors;
optionally, the tumor is selected from bladder cancer, ureteral cancer, renal pelvis cancer, and urethral cancer;
optionally, the tumor is selected from bladder cancer.
